# EUROPEAN PATENT APPLICATION

(11) **EP 4 844 402 A1**
(43) Date of publication of application: **25.11.2026**
(21) Application number: 25177481.6
(22) Date of filing: 19.05.2025
(51) Int. Cl.: G01N 33/38

(54) **A SENSOR SYSTEM FOR MONITORING FOR CORROSION IN REINFORCED CONCRETE STRUCTURES**

(71) Applicant: Technological University Dublin, Dublin D07 EWV4 (IE)
(72) Inventor: BOND, Sean, Dublin D07 EWV4 (IE)
(74) Representative: FRKelly

(57) **Abstract**

The specification provides a sensor probe (301) configured for use in monitoring the state of health of concrete structure. The sensor probe (301) comprises a plurality of sensors (327, 341, 350, 360, 370) and a sensor probe body (320) configured in use to be received in a core void (54) formed in the reinforced concrete, at the test site. The sensor probe body supports the plurality of sensors (327, 341, 350, 360 and 370) to provide for the precise positioning of the sensors relative to the core void (54). The specification also provides for system for monitoring for and identification of corrosion in concrete structures, and for monitoring the test site during remediation.

## Description

### Field

The present invention relates to a system for detecting and treating corrosion in reinforced concrete structures. In particular the system provides for a precision-controlled treatment of corrosion.

### Background

Reinforced concrete structures, such as bridges, buildings, and marine infrastructures, are fundamental to modern society's infrastructure. Over time, these structures may be susceptible to deterioration due to environmental factors, particularly chloride-induced corrosion of the embedded steel reinforcement. In such a situation, chloride ions penetrate the concrete cover, reaching the steel reinforcement and initiating corrosion processes. This leads to structural degradation, reduced load-bearing capacity, and potentially catastrophic failures if not detected and mitigated promptly.

Traditional methods for monitoring the health of concrete structures and mitigating corrosion are often reactive and labour-intensive. They typically involve periodic inspections, visual inspection, manual data collection, and large-scale interventions that can be costly and disruptive.

The present invention aims to address the problems in identifying problems in the health of concrete structures and to provide timely intervention and treatment.

The present invention aims to provide an improved system for monitoring and detecting corrosions and for targeted remediation.

### Statements

The present invention provides an integrated, proactive solution that combines real-time monitoring with targeted remediation, leveraging sustainable energy sources and advanced data analytics. Such a system would enable continuous assessment of structural integrity, early detection of corrosion, and timely intervention to extend the lifespan of critical infrastructure while minimising costs and environmental impact.

The arrangement of the specification and claims aims to provide improved solutions for monitoring for, identifying, and addressing corrosion in reinforced concrete structures.

The system is an innovative, integrated solution designed to proactively monitor and mitigate chloride-induced corrosion in reinforced concrete structures. The system combines advanced sensor technology, a comprehensive software architecture with machine learning capabilities, and electrochemical chloride extraction (ECE) system. In preferred arrangements the electrochemical chloride extraction (ECE) system is photovoltaic (PV)-powered.

The sensor system includes an advanced sensor probe. The sensor probe includes a combination of sensors to provide detection of a variety of relevant parameters. The sensor probe is robust and self-contained for direct installation at target locations that may be remote and difficult to access. The sensor probe is configured to be retro-fitted, includes an integrated power supply and is configured for wireless communication.

According to a first aspect there is provided a sensor probe (301) configured for use in monitoring the state of health of concrete structure. The sensor probe (301) comprises: a plurality of sensors (327, 341, 350, 360, 370) and a sensor probe body (320) configured in use to be received in a core void (54) formed in the reinforced concrete, at the test site. The sensor probe body supports the plurality of sensors (327, 341, 350, 360 and 370) to provide for the precise positioning of the sensors relative to the core void (54).

The sensor probe comprises a plurality of sensors for use in assessing corrosion risk in reinforced concrete. The sensor probe is also configured for use in monitoring corrosion reduction during ECE remediation treatment. The probe is sized and dimensioned to be received in a core void that is formed in the concrete at the test side to receive the sensor probe. The core void also referred to as a concrete void or defines a test hole or void or aperture for receiving the sensor probe that is formed in the concrete for example when a plug of concrete is removed. The sensor probe body supports the sensors. The sensor probe body defines a housing having an interior space for receiving components of sensors. Sensors or components of sensors that do not need to be in contact with the concrete may be located internally within the probe body. The external surface is configured to provide for locating elements of the sensors, as required, for example in contact with the concrete and/or in the core void in proximity to the concrete. The sensor probe comprises a plurality of sensors to allow detection of different parameters which are analysed together in combination to provide a comprehensive monitoring of the test site.

In one arrangement, the sensor probe is configured to be installed in a retro-fit installation at a target site.

The sensor probe comprises a self-contained senor probe having a housing that is configured and formed for installation at a target location or test site in a concrete structure. The procedure for retro-fitting a sensor probe at a selected location includes forming a core void in the concrete structure for receiving the probe and installing the probe. The sensor probe is configured for coupling to a controller and communication system. The system includes plug and play components that allow for easy and flexible installation. The sensor probe is configured to be installed locally at concrete structures, for example in the event that corrosion is suspected or identified or as a precaution to monitor for corrosion. Often the test site may be at a location that is remote or difficult to access on a concrete structure. The sensor probe is configured for easy, flexible and secure installation and for operation over an extended period without the requirement for frequent maintenance. The sensor probe is configured to communicate the sensor data for example by wireless communication to a control system for data analysis. The sensor probe is configured to be controlled remotely including for example to be activated to allow control of frequency of one or more measurements. The sensor probe comprises an integrated power supply such as a battery or PV supply.

According to anther arrangement, the sensor probe further comprises: a sensor probe cap (310). The sensor probe body (320) and cap (310) are arranged about a central longitudinal axis (Z). The sensor probe body (320) comprises a sensor probe housing (324) and an external surface (325), the sensor probe body (320) having an elongated form extending longitudinally (Z1-Z1) from a distal end (322) to a proximal end (321). The sensor probe cap (310) is located at the proximal end of the sensor probe and configured in use to be located externally of the core void (54). The sensor probe body (320) and the cap (310) are configured to provide for precise positioning of each sensor (327, 341, 350, 360 and 370) relative to the core void.

The cap and the configuration and co-operation of the cap and sensor probe allows for sealing to of the sensor probe within the test void, to protect the probe and void from the environment and elements. The sensor probe comprising a plurality of sensors (327, 340, 350, 360, 370), wherein one or more of the sensors (327, 340, 350, 360, 370) or components thereof, are locatable at defined locations relative to the external surface (325) of sensor probe, to allow for precise positioning of the one or more sensors, or components thereof, relative to the test site, including relative to a rebar ( 51-1) and/or the concrete void (54) at the test site.

The sensor probe comprises a probe body having in one exemplary arrangement an elongated cylindrical form. Elements of the one or more of the sensors (327, 340, 350, 360, 370) such as a sensor interfaces or sensor electrodes are located at predefined locations on the sensor body. The location of the sensors and elements of the sensors are defined in terms of axial and/or radial location and/or circumferential positions relative to the elongated body portion (320).

In one arrangement the sensor probe (301) comprises: at least one potential sensor (370) to measure the electrochemical potential difference between the embedded concrete steel reinforcement (51) and a reference electrode (373, 1373), to detect corrosion activity.

The potential sensor (370) is configured to be coupled to the embedded concrete steel reinforcement and the reference electrode.

In one arrangement the potential sensor (370) comprises a Half-Cell potential sensor and the reference electrode comprises a stable half-cell electrode. The reference electrode may comprise for example: an Ag/AgCl reference electrode (373) or Cu/CuSO4 reference electrode (373). The potential sensor (370) is operable to measure the potential difference generated by chloride ion activity compared to the reference electrode.

While in a preferred arrangement the reference electrode comprises an Ag/AgCl reference electrode (373) or Cu/CuSO4 reference electrode, it will be appreciated that a reference electrode comprising other suitable material may be provided. The reference electrode may be provided integrated with the sensor probe. The reference electrode may be provided as an independent and separate reference electrode configured for coupling to one or more sensors. The reference electrode comprises a half-cell electrode.

In one arrangement, the sensor probe (301) comprises a resistivity sensor (360) configured to measure concrete resistivity to assess moisture and ion mobility. The resistivity sensor (360) may comprise a 4-Point Wenner Probe (1360) comprising four electrodes (361) to measure the electrical resistivity of the concrete. In an exemplary arrangement, the electrodes are equally spaced apart on the sensor probe body in a direction of the longitudinal axis of the sensor probe.

In one arrangement according to the first aspect, the sensor probe (301) comprises a chloride sensor (350), wherein the chloride sensor is Chloride Ion-Selective Electrode, CI ISE, sensor configured to measure chloride ion concentration for corrosion risk assessment and to monitor for chloride reduction during ECE treatment.

The CI ISE is configured to measures the potential difference generated by chloride ion activity compared to a stable reference electrode. The stable reference electrode may comprise the reference electrode (373, 1373) of the potential sensor (370). Potential changes reflect chloride concentration, with higher concentrations correlating with increased corrosion risk.

In the arrangement of the claims, the CI ISE sensor is tuned for a potential value to provide for measurement of chloride ion concentration. The arrangement of the specification provides for one or more different tuning settings to provide for different ion measurements and/or detection of other ions within the concrete at a test location.

In one arrangement according to the first aspect the sensor probe (301) comprises one or more of:
(i) a temperature sensor (340) to measure ambient and internal concrete temperature to allow for adjustment of corrosion and resistivity measurements;
(ii) a temperature and humidity sensor (340) to measure ambient and internal concrete temperature and humidity to allow for adjustment of corrosion and resistivity measurements.
The temperature sensor may comprise a thermistor (341).

In one arrangement the sensor probe (301) is configured detect data (305) indicative of state of health (304) of the reinforced concrete at the test site. The sensor probe (301) comprises a plurality of sensors (327, 350, 340, 360, 370) each configured to sense different parameters and properties to support a comprehensive analysis of the state of health (304) of the reinforced concrete at the test site. The detected data (305) includes data measured by a plurality of sensors (327, 355, 345, 365, 375).

In one arrangement, the sensor probe cap (310) comprises a fixing member (314) for fixing the sensor probe in location at the test site, and a sealing member (317) for sealing the sensor probe located in the core void (54) from an external environment The sensor probe cap (310) may further comprise a keying element (312) defining a visual indicator to guide a user to precise location of the sensors at a required depth and/or angular orientation relative to the core void (54) and any structural elements embedded in the concrete structure.

The cap comprises keying feature to guide the user in locating the sensors relative to the embedded concrete reinforcements. The keying element is configured to ensure that the sensor probe 301 and the sensors thereon are inserted with a known angular alignment relative to the void 54. This feature and arrangement can be critical for probes 301 that rely on directional or localized measurements, for example, half-cell potential sensors 370 that need to be oriented toward the nearest steel bar 51.

In one arrangement the sensor probe is configured to be coupled to an ECE treatment system, wherein the data detected by the sensor probe comprising sensed data is provided to a controller to control the operation of the ECE treatment system.

In one arrangement, the sensor probe (301) is configured to be coupled to an electrochemical chloride extraction, ECE, system (400), wherein the sensor probe (301) is configured to provide data (305) for use in identifying corrosion and in controlling the operation of the ECE system (400);
wherein the sensed data includes a plurality of data relating to different parameters as detected by the plurality of sensors, wherein the sensed data is indicative of the state of health of the concrete.

In one arrangement, the sensor probe and ECE treatment system are installable in combination at a target location to detect and treat corrosion. Wherein the sensor probe and ECE treatment system are retrofittable at a target location.

According to a second aspect there is provided a system (100, 200) for monitoring for corrosion and treatment of corrosion in a reinforced concrete structure (40, 50, 51) at a target location, the reinforced concrete structure comprising one or more embedded reinforced bars (51), wherein the system comprises:
at least one sensor probe (301) according to the arrangements of the specification; and
   a remediation module (400) comprising an electrochemical chloride extraction, ECE, system (401) configured to provide treatment and remediation (460) of corrosion at the target location, wherein the ECE system (401) comprises an anode and a cathode, wherein the cathode is defined by an embedded reinforced bar of the concrete structure; and
a controller (230, 500);
   wherein the at least one sensor probe (301) is configured to sense data (305) indicative of the state of health of the concrete and, and to provide the sensed data (305) a controller (230, 500);
   wherein the controller (230, 500) is configured to receive sensed data (305) and to analyse the data (305) and determine whether remediation action is required; and
   wherein, upon determining based on the sensed data (305) that remediation action is required, the controller (230, 500) is configured to activate the ECE system (401) to deliver remediation treatment (460).

Within the overall system 100, the control system or controller 230, 500 may be configured to receive sensor data 305 and to analyse the data 305. In one arrangement, the data is compared with predefined thresholds to determine whether remediation action is needed. If it is determined that the measured data exceeds predefined thresholds, a signal is sent to cause operation of the ECE system is to operate to deliver remediation treatment. The system may comprise control system and on-site monitoring and remediation system, the one or more sensor probes, ECE system and controller may be communicatively coupled. The control system may comprise a controller installed locally at the target location for controlling a locally installed ECE system. The frequency of monitoring may include one or more of real-time, intermittent for example daily or at other defined intervals of time. Different sensors of the monitoring module and of the one or more sensor probes may be set to provide data at different intervals. The monitoring 303 of a state of health 304 of the concrete at the target location may include monitoring the level of deterioration /durability/ chlorine ingress. The ECE treatment 460 includes chloride extraction. Alternatively, the control system 500 may comprise a remote or central data management and control system for central management of a plurality of monitoring and remediation device and test sites, and communication between may be by remote communication or telecommunication.

In one arrangement operation of the ECE system (401) is controllable based on feedback (305) from the one or more sensor probes (301), wherein treatment intensity and duration are dynamically adjustable based on the feedback (305).

The feedback from the one or more sensor probes comprises the data detected by the various sensors and relating to different relevant parameters. The system for example at the controller is configured to analyse the combination of data together, providing a comprehensive analysis.

The system installed locally at the target location provides for a precise and up to date monitoring of the target location. The system is configured to monitor a range of parameters and the sensor data 305 is used to evaluate the state of health 304 and to indicate if treatment 460 is required. The system is further configured to monitor the state of health as treatment 460 progresses and to support dynamic adjust of the treatment as the conditions at the target site change in response to treatment 460. The one or more sensor probes 301 and the controller of the system are configured to provide monitoring 303 at the target location in the reinforced concrete structure over a continuous period of time, including before, during and after operation of the ECE system 401 to allow control of delivery of remediation treatment 460. The system is configured to provide monitoring over a continuous and extended period of time. The monitoring may include sensing of a plurality of parameters in the environment of the sensor at intervals. Different parameters may be measured at different times or intervals of time. The monitoring is implemented in real-time and allows for any changes in the environment or in the parameters under detection to be monitored and for the swift identification of any issues.

In another aspect there is provided an ECE treatment system operable in response to the

### Brief Description of the Drawings

The arrangements of the present application will now be described with reference to the accompanying drawings in which:
Figures 1A and 1B are schematic views of the components of a system for detecting and treating corrosion in reinforced concrete, the system includes a central control system that can be accessed remotely and is configured for communication with modules installed at a local test site;
Figures 2A - 2E provide schematic views of exemplary arrangements of an embodiment of the system of the specification, the system has a modular form; in Figure 2A a system is provided having remote components in communication with a system comprising modules for monitoring and remediation treatment at a test site; in Figure 2B the monitoring module only is installed at a test site; Figures 2C, 2D and 2E show further exemplary arrangements according to embodiments of the specification; Figures 2C, 2D and 2E show details of a remediation module of the system installed at a test side;
Figures 3A, 3B and 3C provide a cutaway plan view from the side and a cross-sectional top view of a sensor probe according to an exemplary arrangement of the present specification;
Figures 4A, 4B, 4C, 4D, 4E, 4F, 4G and 4H provide views of a cap of a sensor probe according to an exemplary arrangements of the specification; Figures 4A and 4B provide top and bottom plan view of a cap of a sensor; Figures 4C, 4D, 4E, 4F and 4G provide a bottom plan view, a bottom perspective view a side plan view, a top plan view and a top perspective view of a cap of a sensor probe according to arrangements of the present specification; Fig. 4H shows a further exemplary arrangement in which the cap comprises a solar receiver 750;
Figures 5A, 5B, 5C are a front plan view, and side and top cross-sectional views of a system according to arrangements of the present specification installed at a target location in a concrete structure including sensor probes and a control unit;
Figures 6A and 6B are a front perspective view and a cross-sectional view of a target site in a concrete structure prepared to receive first, second and third probes of a monitoring module, Figure 6C shows an example of the method of insertion of probes in perspective view; Figure 6B shows an exemplary arrangement of reinforcement bars embedded in the structure and the relative locations of core voids or test voids and sensor probes relative to a reinforced bar;
Figures 7A, 7B, 7C and 7D show perspective, top and cross-section views from the side of an alternative exemplary arrangement of a sensor probe according to an embodiment of the specification installed at a test location and shows an exemplary positioning relative to the reinforced bars; the arrangements shown are similar to that of Figures 5 and 6; in Figures 7 the sensor probe is coupled to an external reference electrode located in a reference electrode void in proximity to the sensor probe;
Figures 8A, 8B and 8C show a top plan view, a cutaway plan view from the side and a cross-section view of the sensor probe arrangement of Figures 7 including an external reference electrode; the arrangement is similar to that of Figures 3 and 4;

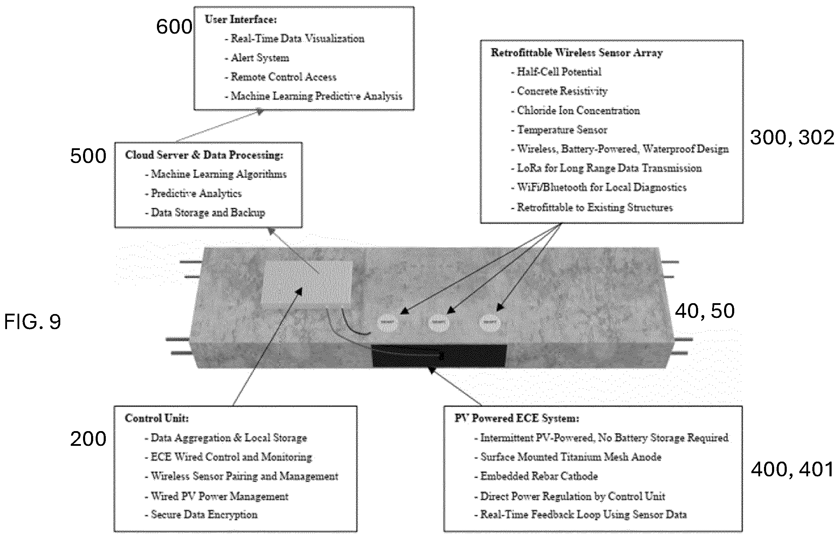

Figure 9 is a perspective and block diagram view of a system 100 according to an exemplary arrangement of the specification;
Figure 10 shows a flow diagram of a method of operating an ECE system according to an exemplary arrangement;

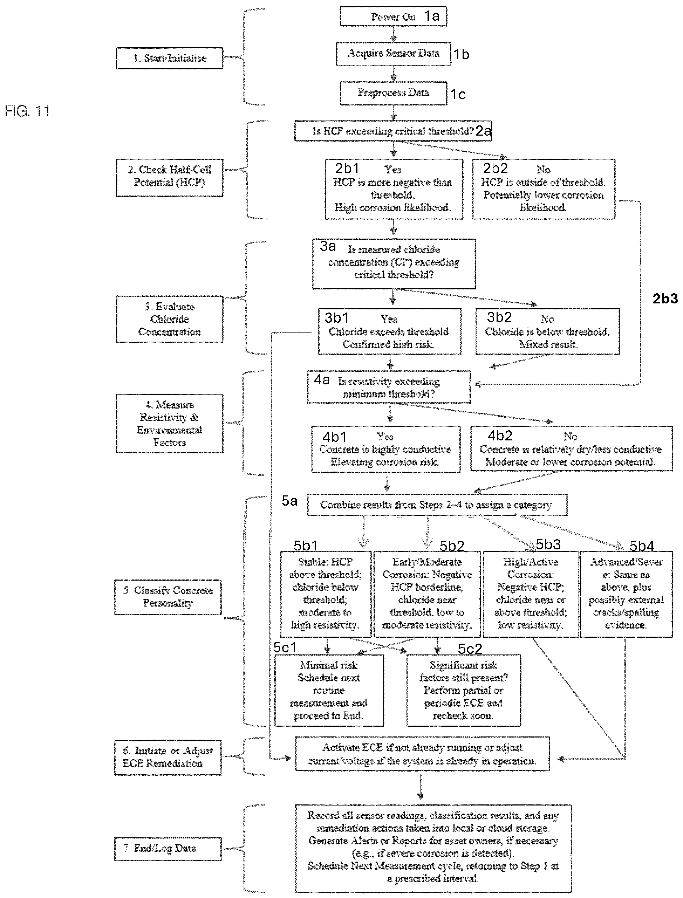

Figure 11 is a flow chart diagram showing operation of a system according to an exemplary arrangement.

### Detailed Description

The present invention will be illustrated using the exemplified embodiments shown in the figures. While this invention has been shown and described with reference to certain illustrated embodiments thereof, it will be understood by those skilled in the art that various changes in form and details may be made therein without departing from the scope of the invention encompassed by the appended embodiments.

The terminology used herein is for the purpose of describing particular embodiments only and is not intended to be limiting of the embodiments of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. Furthermore, to the extent that the terms "includes", "having", "has", "with", "comprised of", or variants thereof are used in either the detailed description or the claims, such terms are intended to be inclusive in a manner similar to the term "comprising".

The present specification relates to a system 100 for controlled monitoring for corrosion in reinforced concrete structures and for providing controlled treatment or remediation of corrosion.

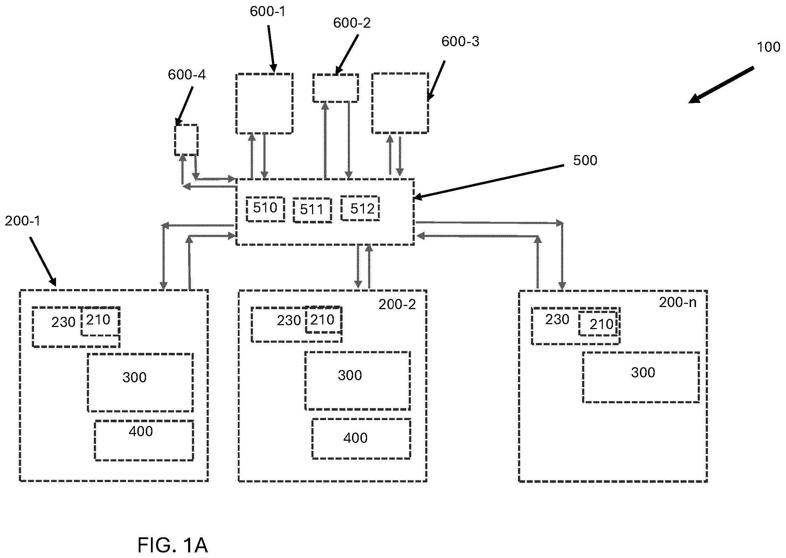

The system 100 is described initially with reference to Figure 1A, which shows a schematic overview of the features and components of the system. The system 100 is configured for monitoring, detecting and treating corrosion in reinforced concrete structures. System 100 comprises a local monitoring and remediation system 200 configured for installation locally on-site at a target location, such as in a reinforced concrete structure 50, 51. The reinforced concrete structure includes one or more embedded steel bars 51, also referred to as rebars. The system in the exemplary arrangement of Figure 1A further comprises a data management and control system 500 and user system 600 that in the exemplary arrangement of Figure 1A are provided remotely from the target location. As shown a plurality of locally installed systems may be coupled to the remote data management and control system 500, located for example in the cloud. The data management and control system 500 can be accessed by a plurality of user systems 600. The arrangement allows for uses to access data from on-site monitoring and/or remediation remotely and to allow control and user inputs and to provide information, data and feedback to users.

In this arrangement a local control unit 230 of the locally installed system 200 may also provide for data management including one or more of data storage, analysis, monitoring, and control of the monitoring and remediation systems. It will be appreciated that the monitoring, storage, analysis and control may be provided locally and/or at the central system 500.

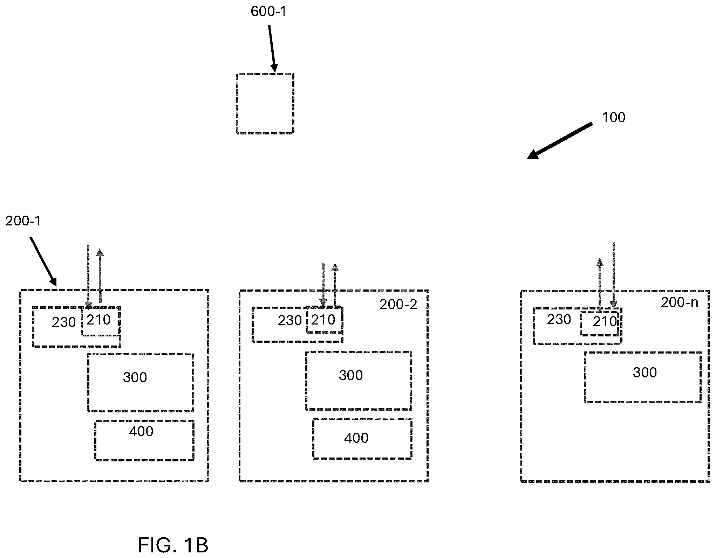

With reference to the exemplary arrangement of Figure 1A, detection, monitoring and analysis of data may be provided locally at control unit 230. It will be appreciated that the management of data and analysis and control may be provided centrally or locally or based on a combination of both. With reference to the exemplary arrangement of Figure 1B, in an alternative arrangement, data management and control is provided at the control unit 230. The control unit 230 may be configured to support direct communication with user devices 600 via communication unit 210.

Referring to Figures 1A and 1B, the locally installed on-site system 200 comprises a sensor module 300 (also referred to as a monitoring module 300) and a remediation module 400. The system further comprises control unit 230. The control unit 230 is configured to be communicatively coupled to the sensor module 300 and to the remediation module 400 for local data management and local control. The system comprises a communication unit 210 for sending and receiving communication signals to and from the data management and control system 500 and/or user device 600. Thus, the local control unit is configured to receive sensor data which may be processed locally and/or communicated to the remote data and control system 500.

The data management and control system 500 comprises a communications interface 510 for receiving and sending communication signals from and to device 200. The data management and control system 500 is further configured for communicatively coupling to a user system 600. The data management and control system 500 comprises a data store 505, a communications interface 510 and processing means 511 and memory 512. Sensor data 305 from the remotely located sensor module 300 is received at data and control system 500. Control signals 505 may be sent from the data and control system 500 to the monitoring and remediation system 200.

User system 600 may comprise a user access system, for example provided in a user computing device. The system 600 is communicatively coupled to the system 500. A user at the computing device using system 600 is able access the data and control system 500. Depending on the configuration of the system the user may be able to access data via system 500 and/or to access data via the local unit 200, 230.

For example, the user may be able to access the data and control system 500 and/or control unit 230 to review data received from one or more on-site system 200-1, 200-2, 200-n, and/or to provide a user input to the system for control of monitoring and/or remediation at one or more of the on-site systems. The connected on-site system 200-1, 200-2, 200-3 may be installed at geographically different locations.

The system 600 may provide access to allow review of historical monitoring data, data relating to progress of remediation, and outcome data. User access may be provided in the form of an APP 610 or another similar user interface 620. The system 600 is configured to allow user input 630 for set-up and/or control of a monitoring and remediation system.

The data and control system 500 and user computing device 600 may be provided integrated. The systems 500 and 600 may be located remotely from the on-site target location. For example, the system 500 may be couped to a plurality of remote on-site target locations and on-site systems 200 and to a plurality of remote user systems 600.

A user input may include inputs to control operation of the on-site system 200, the sensor module 300 or remediation module 400. The data analysis and operations for control of device 200 may be implemented in system 500 and/or in system 600.

While in the exemplary arrangement of Figure 1, the system 100 comprises a monitoring and remediation device 200 in communication with the central data and control system 500 via local control unit 230. It will be appreciated that a plurality of the remote devices 200 may be communicatively coupled to the central data system 500. It will be appreciated that the data and control system 500 may be coupled to one or more user computing devices 600. It will be appreciated that the central data and control system may be provided remotely or may be local, or as a hybrid having local and remote elements.

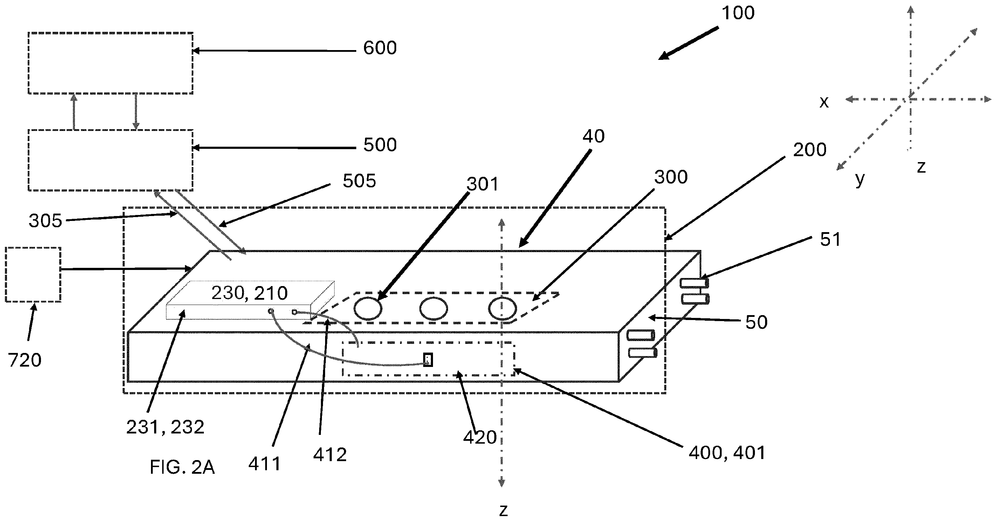

Referring to Figures 2A to 2E, exemplary arrangements of the system 200 installed in various installation configuration and formats at local target sites are described.

The system 200 is configured for installation locally on-site at the target location, such as in a reinforced concrete structure 50 comprising steel bars or rebars 51.

In the arrangement of Figure 2A, the locally installed monitoring and remediation system 200 comprises a sensor module 300 and a remediation module 400. A power source 700 is shown coupled to the system 200. The system 200 further comprises control unit 230 communicatively coupled to both the sensor module 300 and remediation module 400.

The power source may comprise a battery 710. The power source 700 may comprise a solar powered source such as for example a photovoltaic 720. It will be appreciated that other suitable power source arrangements may be provided including a combination of renewable and/or battery arrangements.

The control unit 230 is configured for communicative coupling locally with the sensor module 300 and the remediation module 400. The control unit 230 is configured to receive data 305 from the sensors of the sensor module 300 and to send control signals 306. The control unit 230 is configured to send and receive control signals to and from the remediation module 400. The control unit 230 further comprises a communication unit 210 for communicating with the data and control system 500 and/or one or more user devices 600.

Control unit 230 of an exemplary arrangement comprises a processor 231 and memory 232. The control unit 230 provides a data aggregation and local storage means. As described above the control unit is configured to communication with the data management and control system 500. However, it will be appreciated that in an alternative arrangement the control unit 230 may be configured to provide data analysis locally and to provide control of the remediation module 400 directly and locally. The analysis and control may also be provided based on a combination of both the local and remote systems,

The remediation module 400 comprises an Electrochemical Chloride Extraction, ECE, system 401 that uses Electrochemical Chloride Extraction, ECE, technology to mitigate chloride-induced corrosion in reinforced concrete structures by extracting harmful chloride ions from the concrete matrix and re-passivating the embedded steel reinforcement. The remediation module is configured to provide remediation treatment 460 also referred to as ECE Electrochemical Chloride Extraction treatment 460 at the target site. The treatment is an electrochemical treatment which is provided by operation of the ECE system 401.

The coupling between the control unit 230 and the ECE system 401 may comprise wired connections 420, 421. The control unit 230 is configured to provide a control of operation of the ECE system 401, including one or more of power regulation control, control of operation, control of operation schedule, and control of operation based on feedback from the sensor module 300. The control unit 230 may be further configured to provide control of power management for operation of the ECE system 401 of the remediation module 400. As described above the remediation module may be coupled to a PV power source 720, the control unit 230 is configured to control operation and power management. The PV power management by the control unit 230 may be based on a wired coupling 721, 722.

The control unit 230 is configured for wireless sensor pairing and for management of the sensor module 300 coupled thereto. The control unit 230 is further configured to provide data communication to and from the coupled data and control system 500. The control unit 230 may comprise secure data encryption.

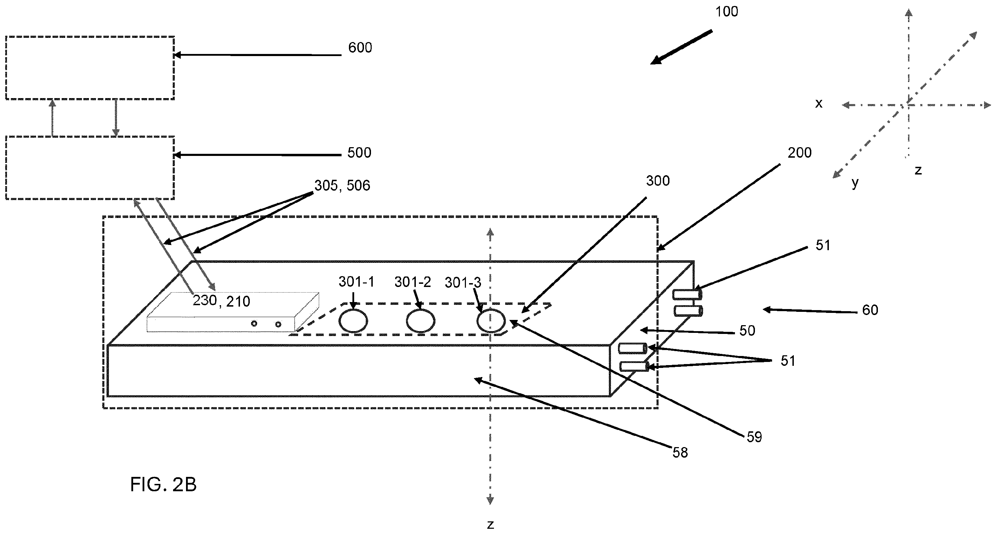

Figure 2B shows an exemplary arrangement according to an embodiment of the invention of an installation of system 200 locally on-site in which the sensor module 300 only is installed. The sensor module 300 can be provided separately and independently from the remediation module 400. For example, a user may select to initially install the sensor module 300 and then if the monitoring indicates detection of an issue, it would be possible to later install the remediation module 400 as a further module of the same overall system. The local system 200 is configured as a modular system and as such the local on-site installation of system 200 can be varied and tailored to the required applications and can be changed over time and as circumstances change. The on-site system 200 can be extended if needed for example by addition of the remediation module 400. It is also envisaged that after remediation is completed it would be possible to remove the remediation module 400 while maintaining the sensor module 300 in place for a period of post-treatment monitoring. The sensor module 300 and the remediation module 400 are but configured for plug and play connection with the system 200 and controller 230.

The system 200 comprising the control unit 230 and sensor module 300 may in an exemplary arrangement comprise battery power sources. In addition, or alternatively the sensor module may comprise a solar power module, for example a photovoltaic module.

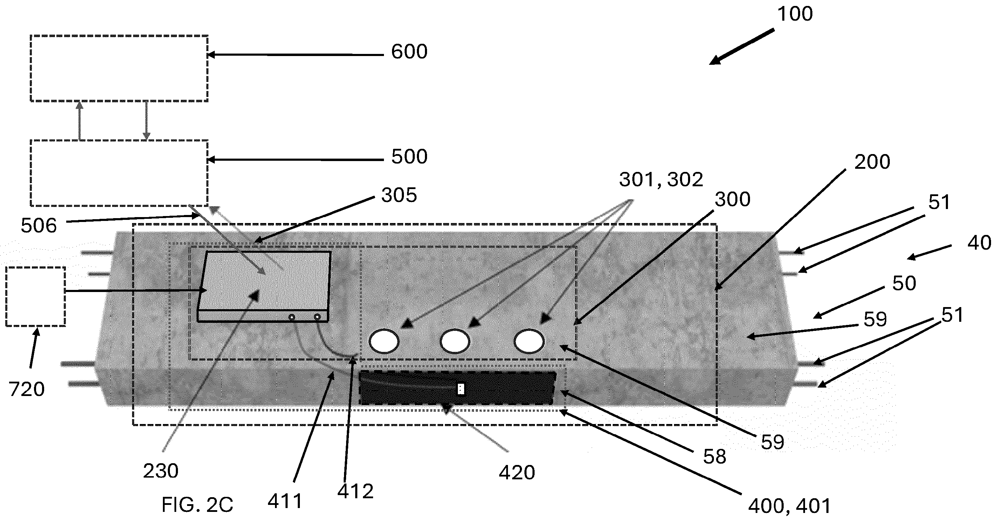

Referring to Figure 2C, in the exemplary arrangement the local system 200 further comprises a remediation module 400. The remediation module 400 of the exemplary arrangement comprises a solar power source 720.

Thus, it is envisaged that the system 200 when operating for monitoring based on the installation including the sensor module 300 only may use a compact power source such as a battery and/or a photovoltaic source. When the system 200 is provided in a full installation and configured for operation of both the monitoring and remediation modules, then then installation may include the additional PV power source 720 arrangement for providing power for the remediation module 400 and optionally other system components.

Further the system 200 is advantageously configured to be installed in a retro-fitting installation. For example, the system does not require embedding internally within a concrete structure when it is being constructed. If at some stage after a concrete structure has been built, it is decided or required to monitor for corrosion in the reinforced concrete it is possible to retro-fit the system 200 locally at an on-site target location. The installation method is described further later. When the system 200 is installed at a target location as part of the system 100 is configured to connect to the network comprising the system 100 and data and control system 500 and user system 600. Thus, with a local retro-fitting installation of system 200 the user automatically has access to monitoring data for assessing whether remediation is required and for control of the local installation.

It would also of course be possible to install the system 200 during construction of a reinforced concrete structure 50, 51.

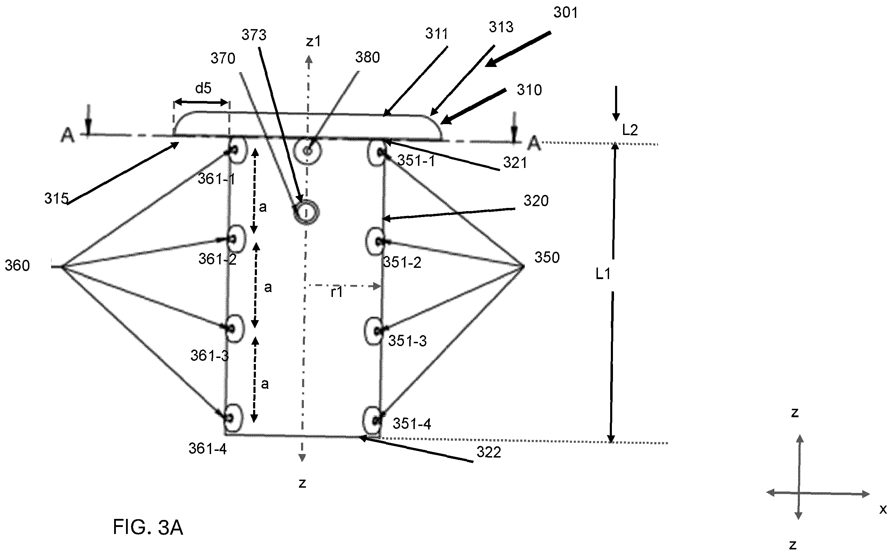

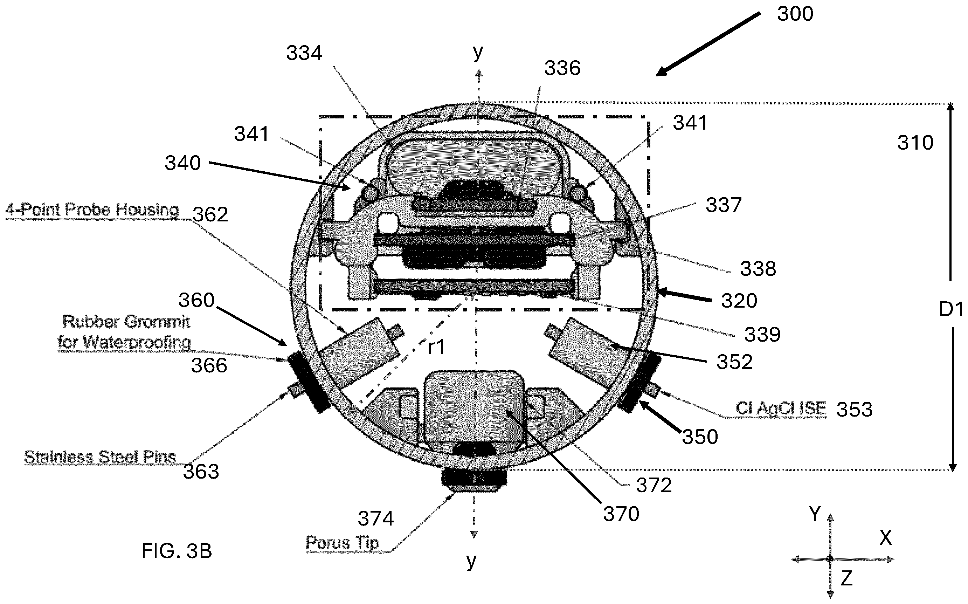

Referring to Figures 3, sensor probe 301 is described in further detail. The monitoring module 300 comprises a sensor array 302 comprising two or more sensor probes 301. The monitoring module 300 is configured to provide monitoring 303 of the target site and to deliver sensor data 305 to the control unit 230 and to the system 500. The control unit 230 is configured to provide control signals 306 to the monitoring module.

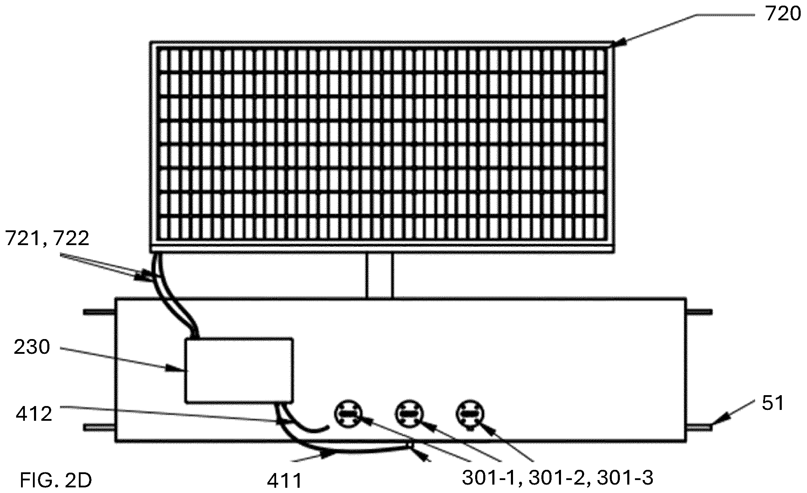

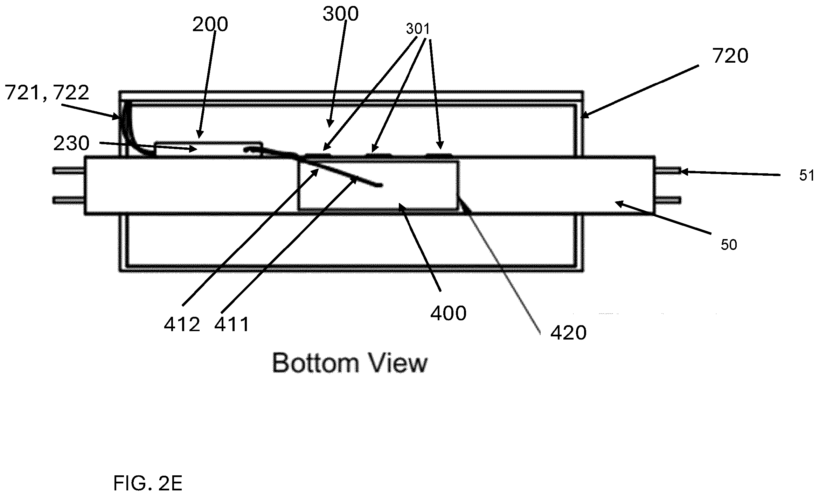

In the arrangement of Figures 2, the sensor array 302 comprises three sensor probes 301-1, 301-2, 301-3.

The sensor array 302 comprises a retrofittable sensor array 302. The sensor array further comprises a wireless sensor array 302, each of the sensor probes is configured to communicate via wireless communication with the control unit. As described above the monitoring module 300 is communicatively coupled to the control unit 230. Sensor data 305 is communicated from the sensor modules 301 of the sensor array 302 to the control unit 230.

Each sensor probe 301 comprises a plurality of sensors 327 configured to provide the measurement of data, including different data parameters and to provide the sensor feedback of measured data 305 required for monitoring 303 of the target site 50.

The sensor probe 301 has a longitudinal axis Z1. The sensor probe 301 comprises a sensor module cap 310 located at a proximal end 313. The sensor probe 301 comprises a sensor probe body 320 that extends about the longitudinal axis from a probe body proximal end 321 near the cap 310 to a distal end 322. The sensor body comprises a sensor wall 323. The body 320 has a longitudinal extent L1 and a radius r1. In the arrangement of the drawings the sensor body 320 has a generally cylindrical form. The sensor wall 323 is arranged extending circumferentially about the longitudinal axis Z1 and defines an internal sensor housing 324 and an external outwardly facing surface 325 of the sensor body.

The sensor probe 301 comprises sensors 327 and related electronic components and power source for control of operation of the sensor probe 301. As shown in the Figures, some of the sensors 327 and electronic components are located internally within the sensor housing 324. Some of the sensors 327 are located having components arranged at the external surface 325 or projecting outwardly relative to the external surface 325 of the sensor body. For example, an interface of a sensor 327 of the probe may be located at the surface 325 to allow contact between the sensor and the surrounding concrete or to locate the interface in proximity to the surrounding concrete. Thus components, for example an interface of a sensor may be located at the surface 325 of the sensor probe body 320 to provide for their positioning, as required, relative to the concrete structure. Components of a sensors of the plurality of sensors 327 may also located at a required distance from other components thereof and at the required separation from other sensors 327 of the senor probe 301.

The body portion 320 of the sensor probe 301 is configured to be located within a concrete void 54 at the target location 50. The form and dimensions of the sensor probe body 320 are provided to correspond with those of the void 54 and configured to provide the necessary spacing, and/or contact and/or interactions between the sensors and the internal wall of concrete void. The void 54 is formed taking account of the form of the sensor probe 301 to be installed therein. The cap portion 310 is configured to be located externally of the concrete void 54 at an external surface 59 of the concrete structure.

In the exemplary arrangement of Figure 3B the sensor probe 301 comprises a battery 334. The sensor probe 301 may further comprise a charging module 336. The sensor probe 301 further comprises an internal electronics housing 338 and a micro controller 337 and an analog to digital converter 339.

As noted above the sensor probe 301 of Figures 3A and 3B comprises a plurality of sensors 327 for sensing various data measurements and parameters. Further details of different sensors of the sensor probe 301 of the exemplary arrangement of Figures 3 are provided below.

The sensor probe 301 comprises a half cell potential sensor 370. The half-cell potential sensor 370 in the exemplary arrangement comprises an Ag/AgCl reference electrode 373. The half-cell potential sensor 370 is configured to detect corrosion activity by measuring the electrochemical potential difference between the embedded concrete steel reinforcement 51 and the Ag/AgCl reference electrode 373. The Ag/AgCl reference electrode provides a stable half-cell potential measurement, representing the electrochemical potential of the embedded steel reinforcement 51, with a standard reference potential of approximately +199 mV versus the Standard Hydrogen Electrode (SHE).

The sensor 370 is configured to detect early signs of corrosion by identifying anodic and cathodic zones on the reinforcement surface. The sensor 370 provides data measurements 375 via the control unit 230 to the data system 500. In use and operation of the sensor 370, data measurements 375 comprising a more negative potential, typically below -350 mV versus Ag/AgCl, correlates with and is indicative of active corrosion, while values above -200 mV generally indicate passive corrosion.

The half-cell potential sensor 370 of the monitoring module 300 in operation provides data 375 that is useable by the system 500 to determine when the corrosion state has shifted from active to passive. The sensing and data measurements 375 also support optimisation of the ECE /remediation duration and guide for voltage adjustments in the operation of the ECE.

Measurement data 375 is captured by measuring the potential difference between the reference electrode 373 and the embedded reinforcement 51. The data 375 sensed by the sensor 370 comprises the electrochemical potential difference between the embedded concrete steel reinforcement 51 and the Ag/AgCl reference electrode 373.

The sensor 370 comprises sensor housing 372 and a porous tip 374.

The probe 301 comprises an ADC 339 for digitising the data and micro-controller 337 for processing the data. In an exemplary arrangement, the data is digitised using an ADS1115 ADC 339 and processed by an Arduino ESP32-S3 microcontroller 337.

The sensor probe 301 further comprises a concrete resistivity sensor 360. In the exemplary arrangement of Figures 3 the concrete resistivity sensor 360 comprises a 4-Point Wenner Probe 360. The concrete resistivity sensor 360 is configured to measure concrete resistivity data 365 to assess moisture and ion mobility. The moisture and ion mobility are critical indicators of corrosion risk and treatment efficiency during ECE remediation treatment 460.

The Wenner probe 360 comprises four electrodes 361-1, 362-2,361-3 and 361-4 to measure the electrical resistivity of the concrete. The four electrodes are arranged in a line between the proximal end 321 and distal end 322 of the probe body 320. The electrodes 362 are equally spaced apart by a distance "a" over the longitudinal extent L1 of the probe body 320. The probe 360 is configured to be operated by application of an AC current through the outer probe electrodes (361-1, 361-4) while the voltage drop between the inner probe electrodes (361-2, 361-2) is measured. The resistivity is calculated using the formula: *ρ =* 2*πaIV* Where, ρ = resistivity (Ωm), a = probe spacing (mm), V = measured voltage drop (mV), I = applied current (mA).

Concrete resistivity data 365 is used in the system 100, 500, 200 to provide an indirect insight into moisture saturation and chloride ion penetration.

In use of the system 100 and/or the sensor probe 301 and the sensor 360, typical ranges of measurement data 365 for concrete resistivity and corrosion risk assessment include the following. Measurement data 365 of concrete resistivity of above 200 Ωm is indicative of dry concrete with low corrosion risk, measurement data 365 of concrete resistivity 100 - 200 Ωm is indicative of moderate moisture and potential chloride presence. Measurement data 365 of concrete resistivity below 100 Ωm, is indicative of high moisture content and elevated corrosion risk.

Each of the parts of the four-point Wenner probe sensor 360 comprises a sensor housing 362 and an electrode 361. In the arrangement of Figure 3B. the sensor 360 further comprises a rubber grommet 366 for water proofing and a stainless-steel pin 363.

The Wenner probe 360 is configured to monitor resistivity changes during ECE treatment, to allow for monitoring to ensuring sufficient ionic flow through the concrete.

A drop in the resistivity in the measurement data 365 would indicate increased moisture and ionic mobility, which improves chloride extraction efficiency.

### Example 1

In an exemplary arrangement of the sensor 360 of the sensor probe of Figures 3, the AC current source is generated by an AD9833 programmable waveform generator, delivering a 1kHz sinusoidal signal. Voltage measurements are amplified using an INA128 precision differential amplifier for accurate low-voltage detection, then digitized by a 16-bit ADS1115 ADC 339 for high-resolution data capture. An ESP32- S3 microcontroller 337 manages signal generation, data acquisition, and resistivity calculations. However, it will be appreciated that other suitable electronics arrangements may be used.

The sensor probe 301 further comprises a chloride ion concentration sensor 350. In the exemplary arrangement, the chloride ion concentration sensor 350 comprises a Chloride Ion-Selective Electrode, Cl ISE, 351. The chloride ion concentration sensor 350 is configured to measure chloride ion concentration for corrosion risk assessment during monitoring, and to monitor chloride reduction during ECE treatment.

The CI ISE measures the potential difference generated by chloride ion activity compared to a stable Ag/AgCl reference electrode. Detected changes in potential changes reflect chloride concentrations. Higher concentrations correlating with increased corrosion risk. In practice at a target location, Chloride ion accumulation significantly accelerates the corrosion process by breaking down the passive oxide layer that naturally forms on steel reinforcement, exposing it to electrochemical reactions that promote corrosion.

Each of the parts of the Chloride Ion-Selective Electrode 350 comprises an electrode 351 and a housing 352 and a CL AgCL ISE pin 353.

In reinforced concrete, chloride-induced corrosion risk increases when chloride concentrations near the steel surface exceed a critical threshold. Generally, a chloride content above 0.2% by weight of cement (or approximately 0.03% by concrete mass) is considered the threshold for corrosion initiation. Monitoring chloride levels allows for early intervention before corrosion begins, enabling proactive remediation strategies such as electrochemical chloride extraction (ECE) to prevent structural damage. Continuous chloride monitoring also helps assess treatment effectiveness by verifying chloride reduction during ECE treatment cycles.

During remediation treatment 450/ ECE treatment, the use of the monitoring system 200 and sensor 350 to obtain real-time chloride ion measurements 355 allows for dynamic adjustment to the ECE process 460 provided by the coupled ECE system 401. This provides for improved remediation treatment 460 as during operation it is possible to ensure effective chloride removal while avoiding overtreatment.

The chloride ISE 350 setup in the exemplary arrangement of Figures 3 includes glass/polymer body electrodes 351-1, 351-2, 351-3, 351-4 paired with an Ag/AgCl reference electrode 373. Signal amplification is stabilized using an LMP7721 low-noise op-amp, while a CD4051B multiplexer sequentially cycles through the three ISE inputs. The conditioned signals are digitized using a high-resolution ADS1115 ADC 339, with data acquisition and transmission managed by an ESP32-S3 microcontroller 337 for further analysis. It will be appreciated that details of the set-up may be varied without departing from the scope of the invention.

The sensor probe 301 further comprises a temperature sensor 340. In the exemplary arrangement of Figures 3, the temperature sensor 340 comprises thermistors 341 to provide temperature measurement data 345. The temperature sensor is configured to measure ambient and internal concrete temperature to adjust corrosion and resistivity measurements. Thermistors 341 measure temperature-dependent resistance changes. At the target site temperature affects corrosion rates. At the target site temperature also affects chloride ion mobility. The temperature sensor detects temperature data and provides measured temperature data 341. In practice at a target site higher temperatures accelerate corrosion activity and ionic movement, while lower temperatures slow down both processes.

Monitoring of temperature data 345 during remediation treatment 406 also provides useful feedback for control of the treatment 406. Temperature data 345 may be factored into the resistivity and chloride measurements, providing for improved accuracy of ECE treatment optimisation and corrosion risk assessment.

In an exemplary arrangement according to Figures 3 the temperature sensor 340 setup uses 5 kΩ Negative Temperature Coefficient (NTC) thermistors paired with a 5.1 kΩ fixed resistor in a voltage divider circuit. The voltage across the divider varies with temperature, providing a predictable nonlinear response. This signal is measured by the ESP32-S3 microcontroller's 337 analogue input, where it is converted to temperature values using the Steinhart-Hart equation for improved accuracy. It will be appreciated that the details of the exemplary set-up may be varied without departing from the scope of the invention.

The Ag/AgCl half-cell sensor 370 is shared as the reference for both half-cell potential measurements 375 and chloride measurements 355, ensuring consistent readings. Temperature sensors 340 influence the calibration of both resistivity 360 and chloride sensors 350 due to temperature-dependent electrochemical behaviour.

The sensor probe 301 of the monitoring system 300 is configured for wireless communication. The sensor probes 301 of the array 302 are configured to provide measurement data 305 to the control unit 230 and via to the data and control system 500 of the system 100. The measurement data 305 provided to the control unit 230 includes data 375, 365, 355, 345 from one or more of the sensors 370, 360, 350 and 340 of the plurality of sensor 327.

The monitoring module 300 and sensor probes 301 are configured for a local data access, and communicative coupling with control unit 230, and for diagnostics. The monitoring module 300 and sensor probes 301 are configured to be battery-powered. The battery power 334 arrangement provides for flexible installation and long-term operation in remote locations. The battery 334 may comprises for example a 3.7V LiPo battery.

The sensor probe 301 is configured to be waterproof when installed. The sensor probe 301 is configured such that when it is located in position internally within a concrete void 54 it is sealed from the external environment.

The sensor probe further comprises a port 380 comprising an inlet hole for receiving an embedded reinforcement wire. The port 380 is provided to allow connection between the sensor probe and a rebar 51 that is located in proximity to the probe 301.

Electrodes of the half-cell potential sensor 370, the concrete resistivity sensor 360 and the chloride ion concentration sensor 350 are all surface mounted electrodes which are located on the external surface 325 of the sensor probe body 320. That is electrode components of each of these sensors are arranged at the surface of the body 320 to allow direct interfacing with the test environment. The components are externally mounted to allow their positioning as required relative to the concrete 50 and/or the rebar 51 at the target site.

The surface mounted electrodes and components of the sensors may be resiliently mounted to the surface of the probe body, as required to provide for a positive contact with the concrete. The probe is configured for a friction fit within the concrete void. The probe body may comprise one or more engagement features for engaging with the concrete void for example one or more resilient members that compress as the probe is being inserted and expand to hold the probe in position in location when it has been installed.

Other components of the sensor probe for example the thermistors 341, battery and electronics are located internally within the probe housing 324.

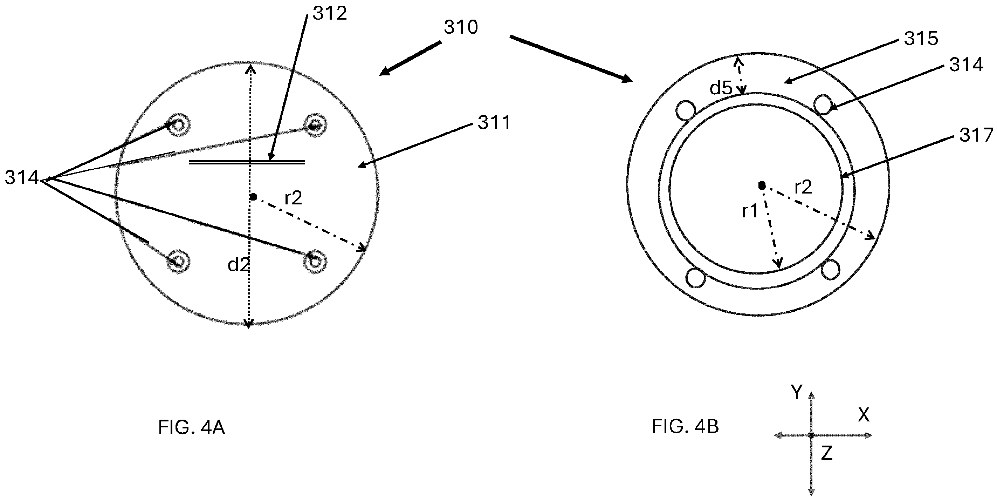

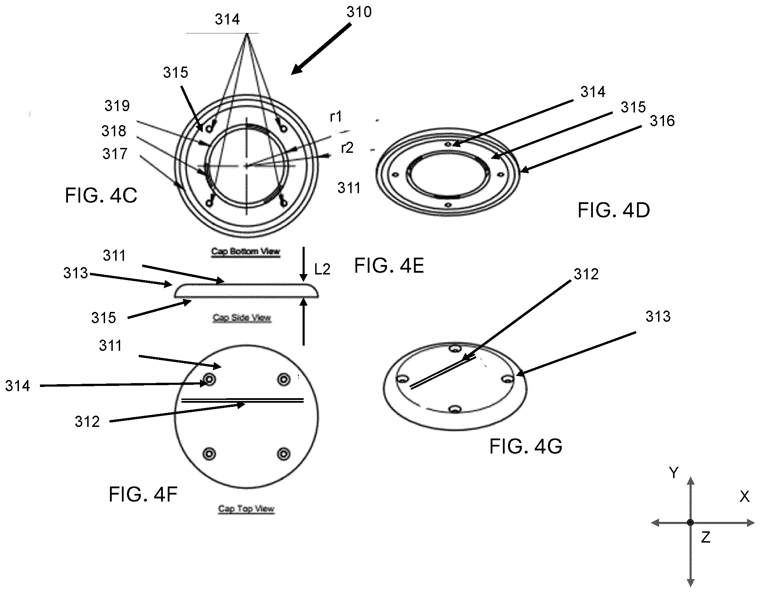

The cap 310 of the sensor probe 301 is described with reference to Figures 4A and 4G. As shown the cap 310 is located at a proximal end of the probe 301 and is configured to be located externally of the concrete core void 54 when the probe 301 is installed at a target location. The cap comprises an external outwardly facing upper surface 311 and a lower surface 315 defining a rim 316 that extends outwardly in a radial direction relative to the central longitudinal axis Z1 a distance d5 relative to the external surface of the probe body 320. The cap has a radius r2 which is greater that the radius r1 of the probe body, r2-r1=d5. The cap 310 in use when installed externally of the concrete void provides has a footprint greater than that of the probe body or void and provides an extended area of cover over the proximal end of probe body located within the core void. The cap 310 has a depth in the longitudinal direction Z1 of L2.

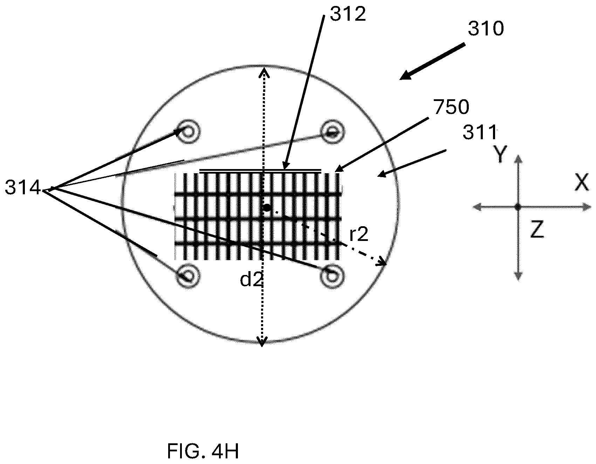

The cap 310 as shown in Figures 4 in a plan view has a circular form and radius r2. The cap comprises apertures 314 located spaced apart in each quadrant and in the area radially external to the probe body that is located distal to the cap 310. The apertures 314 are through holes for receiving fixing elements such as screw or pin to securely fix the cap 310 and the probe body 320 relative to the core void 54.

The cap 310 further comprises one or more keying features 312. In the exemplary arrangement keying feature 312 comprises a line indicator that in Figure 3C is arranged having a horizontal orientation. The keying features 312 are configured to provide to the user an externally visible indication of the orientation of the sensor probe 301 and body 320 relative to the core void 54.

The keying feature 312 thus allows for the probe sensor to be positioned as required relative to the rebar 51 that is embedded within the concrete. For example, this would allow for locating of one or more of the sensors to be located facing towards and in proximity to a rebar 51, as required.

In a further exemplary arrangement, the radius r2 of the cap 310 may be configured to provide a visual indication of the location of rebars 51 relative to the probe body. The diameter of the cap 310 may correspond to the distance between adjacent rebars 51-1, 51-2. The assist in locating the probe centrally between the rebars 51-1, 51-2 and provide a visual guide to the user as to the underlying and non-visible structure 50.

The sensor cap 310 is configured not only to seal the concrete void 54 but also to facilitate precise orientation of the sensor probe 301 relative to the internal reinforcement rebar 51. The cap 310 features a keying element 312, may comprise a marker or other element such as a protrusion or indexing notch. The keying element is configured to ensure that the sensor probe 301 and the sensors thereon are inserted with a known angular alignment relative to the void 54. This feature and arrangement can be critical for probes 301 that rely on directional or localized measurements, for example, half-cell potential sensors 370 that need to be oriented toward the nearest steel bar 51. In some implementations, the circumferential dimension r2 of the cap's outer rim correlates with the known concrete cover over the steel reinforcement 51 with respect to the sensor housing 324 outer diameter or the perpendicular distance from the steel to the closest surface of the void 54. For example, the radius r2 of the cap 310 can serve as a guide for where the concrete void 54 should be placed to situate the sensor probe 301 and sensors thereon in a region most susceptible to corrosion or chloride ingress. The keying element 312 and cap radius r2 thus work together to ensure repeatable, accurate placement of the sensing elements of the sensor 327 and to create a low-profile, flush finish on the concrete surface once installed.

The cap 310 may further comprise a seal locator channel 317 located on the lower surface 315 and recessed relative to the lower surface 315. The seal locator channel 317 may be arranged extending circumferentially about the cap located between apertures 311 and the probe body 320. When the cap 310 is fixed to the concrete, a seal may be located in the seal locator channel 317 to provide a water proof seal between the cap 310 and the concrete and surrounding probe body 320. The seal may comprise an O-ring type seal or other compressible seal.

In an alternative arrangement sealant may be located between the lower surface of the cap 315 and the concrete surface 58 when the cap is being fixed to the concrete surface, in this alternative arrangement the cap 310 does not comprise a seal locator channel.

It will be appreciated that while an exemplary sealing arrangement has been described a different configuration of seal may be provided without departing from the scope of the invention.

In a further exemplary arrangement according to Figure 4H, the cap may include a solar panel 750 configured to provide power for operation of the sensor probe. The solar module at the panel 750 absorbs light from the sun during the day and converts it into electrical energy via the photovoltaic effect. The solar panel may be used to provide power in co-operation with a battery or as an alternative. The sensor probe may include both a battery and a solar panel which can be used selectively or together as appropriate.

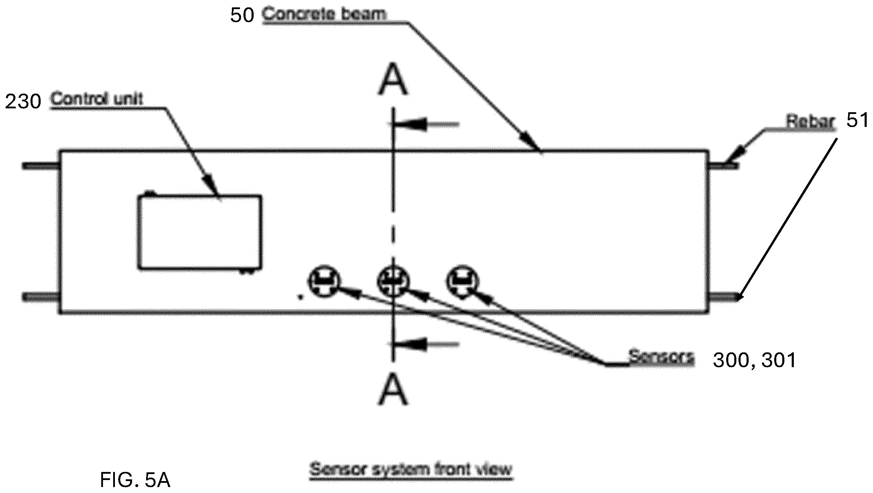

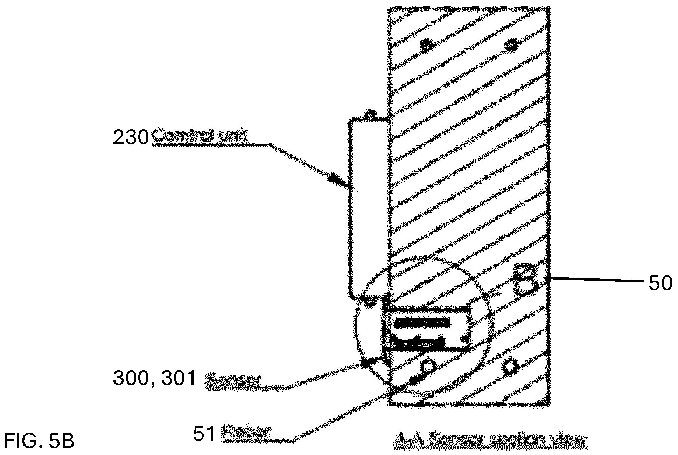

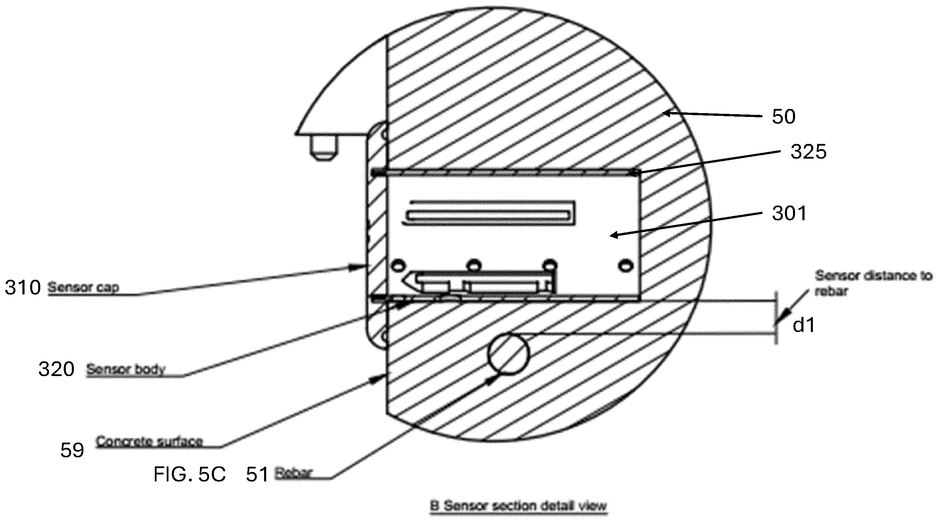

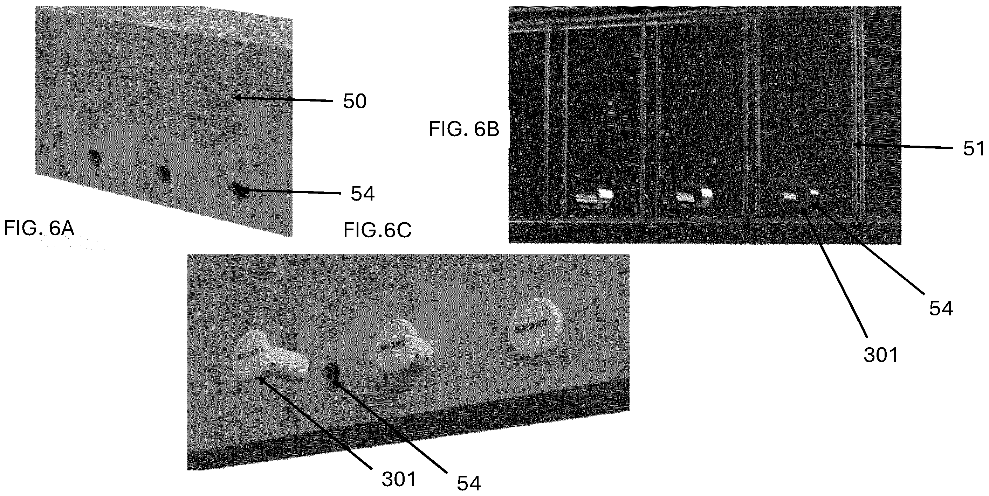

Referring to Figures 5, sensor probe 301 is shown installed at a target location in a concrete structure or beam 50 comprising rebar 51. A array of sensor probes 301 are installed in communication with control unit 230. The one or more sensor probes are located in proximity to a rebar 51 at a separation d1. Referring to Figures 6 a example target location and installation of sensor probes is shown.

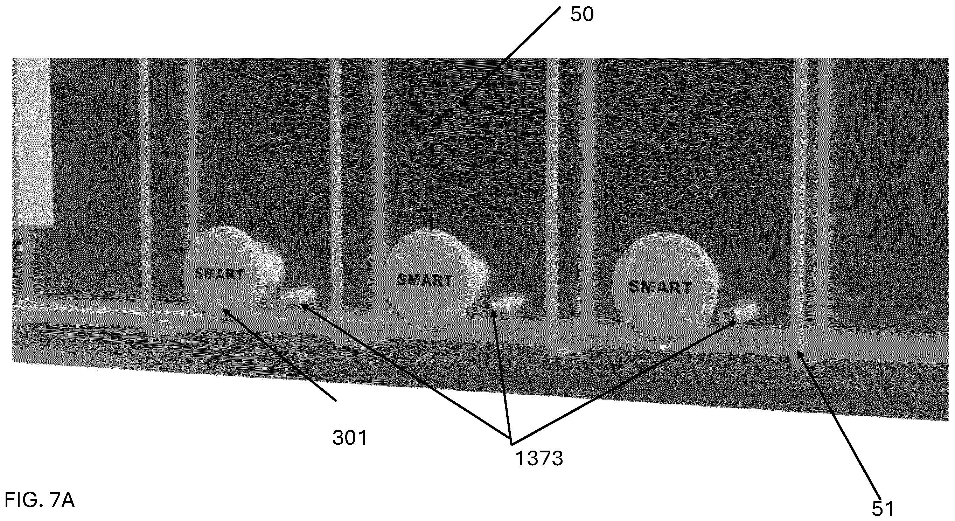

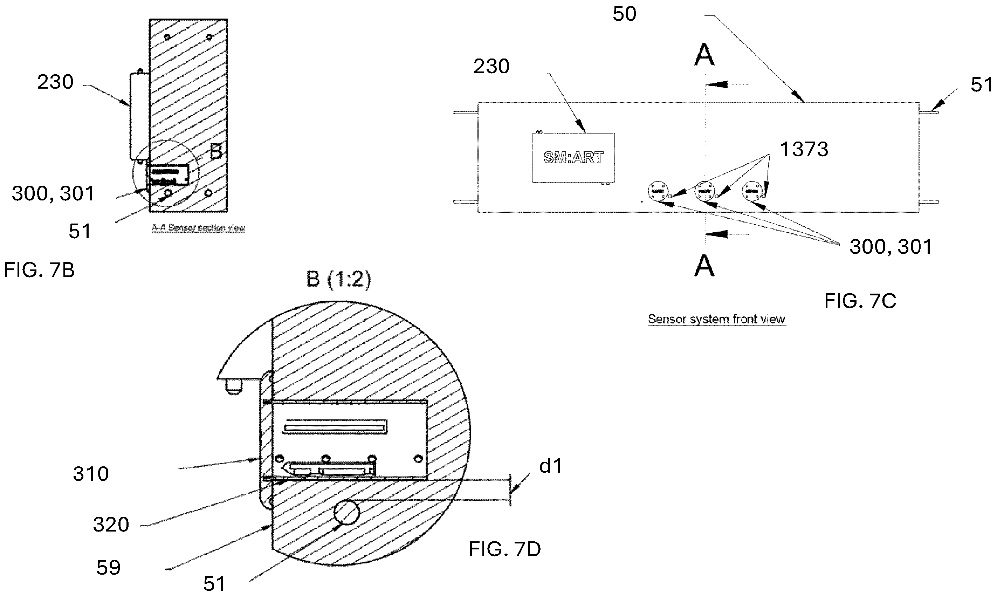

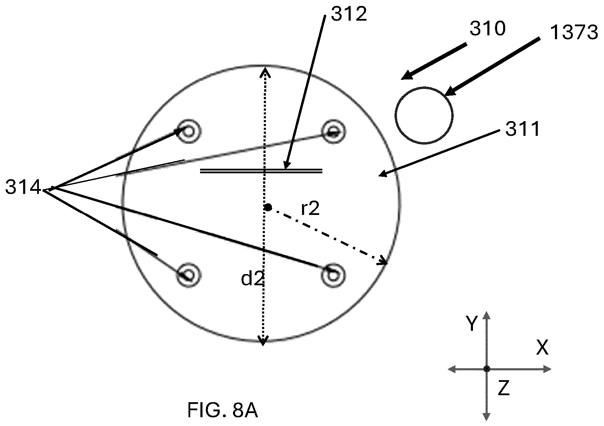

While in the arrangement of Figures 3 and 6, the sensor probe 301 includes a reference electrode 373, it will be appreciated that in an alternative arrangement such as shown in the exemplary Figures 7A to 7D, and 8A to 8C an external reference electrode 1373 may be provided for coupling to the sensor probe as required. The external electrode 1373 is a half-cell electrode similar to the reference electrode 373. In Figures 7, the external reference electrode 1373 is located in a separate void formed in the concrete in proximity to the probe 301 and test void formed to receive a sensor probe. The external reference electrode 1373 is configured for coupling to the sensor probe 301. One or more connections are provided between the reference electrode 1373 and the probe 301. The probe 301 also comprises an inlet hole 380 for receiving a connection to an embedded rebar.

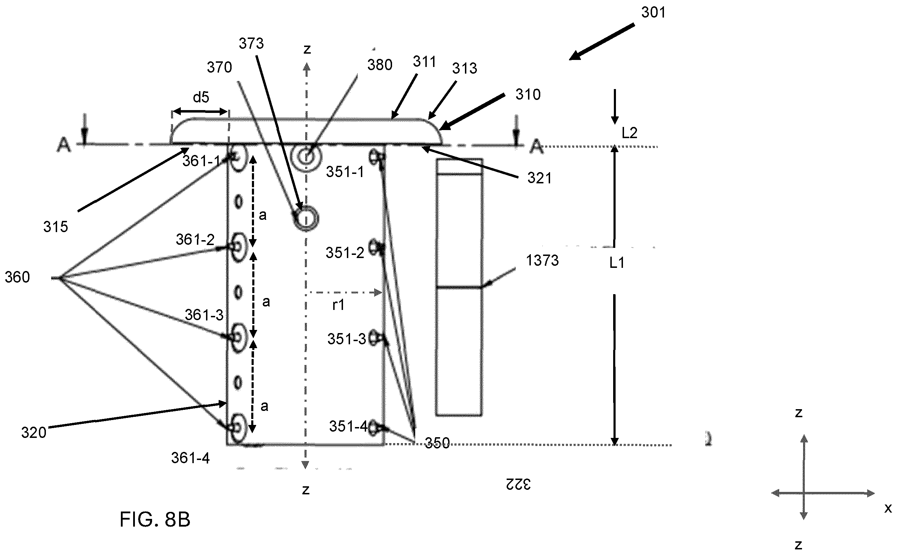

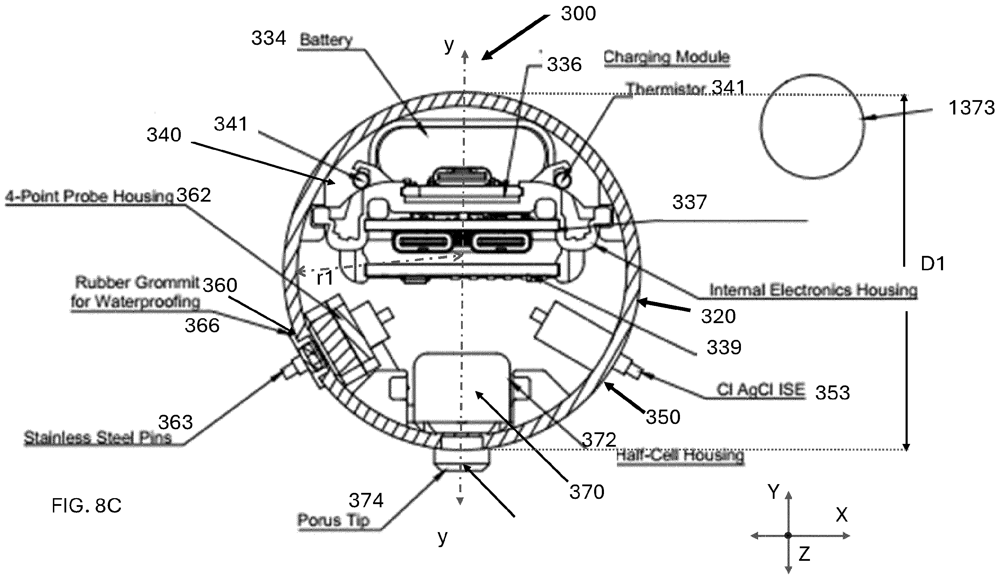

The probe of Figures 8 is similar to that of Figures 3 and 4 and the same reference numbers have been used where appropriate.

In an exemplary arrangement, the sensor 370 comprises a Half-Cell potential sensor and the reference electrode comprises for example: an Ag/AgCl reference electrode 373, 1373 or a Cu/CuSO4 reference electrode 373, 1373. The sensor is operable to measure the potential difference generated by chloride ion activity compared to the stable reference electrode. The reference electrode 373, 1373 may also be coupled to the Chloride Ion-Selective Electrode 350.

Referring to Figures 2D and 2E the remediation module 400 is described in further detail.

The remediation module may be installed as part of the system 200. The remediation module is located at a target site on a reinforced concrete structure. The drawings indicate a target site 50 of a concrete structure 40, reinforced steel bars 51 also referred to as rebars 51, a first surface 58, a second surface 59.

The remediation platform of the system 100 and remediation module 400 are based on an Electrochemical Chloride Extraction, ECE, technology and system 401. The ECE system 401 and operation thereof is directed to mitigating chloride-induced corrosion in reinforced concrete structures by extracting harmful chloride ions from the concrete matrix and re-passivating the embedded steel reinforcement 51.

The system 401 is configured to provide an externally applied electrochemical treatment 460 that uses a controlled current 480 to drive chloride ions towards a positively charged anode 420 and away from the steel reinforcement 51.

The system 401 comprises an anode 420. The anode 420 is mounted in proximity to the steel reinforcement structure 51 at a surface 58 of the concrete structure 50. The anode 420 is configured to be operated as a positively charged anode under application of a controlled current 480. The anode 420 comprises a mesh anode. In the exemplary arrangement of Figures 2, the anode 420 comprises a Titanium mesh anode. It will be appreciated that alternative anode arrangements may be used without departing from the scope of the invention. However, as described in the exemplary arrangement a Titanium mesh anode 420 has been used and the arrangement provides advantages.

The anode 420 is provided as the external electrode that is mountable to the surface 58 of the concrete and configured in use to deliver a positive charge at the concrete surface 58 during ECE treatment 460 i.e. during operation of the remediation system 401.

The anode mesh 420 (for example Titanium mesh anode) acts as the primary current distributor in the electrochemical treatment process 460. The anode mesh 420 is configured to provide a uniform current distribution across the concrete surface 58. This is turn maximises the chloride extraction efficiency of the process 460.

In the exemplary arrangement of Figure 2C, 2D and 2E, the anode mesh 420 comprises a Titanium mesh anode which advantageously has a high corrosion resistance and long-term durability in electrochemical systems.

In the installation of the remediation module 400 the ECE system 401 further comprises a cathode 430. The cathode 430 of the ECE system 401 comprises the embedded rebar 51-1. The rebar 51-1 is configured to act as a protected cathode 430 in the electrochemical process 460, for passivating the steel surface. The embedded steel reinforcement 51-1 serves as the negative terminal during the ECE treatment 460. As the system 401 is operated, electrons supplied to the rebar 51-1 reduce the electrochemical activity, shifting the steel towards a passive state while repelling negatively charged chloride ions toward the anode 420. The anode is mounted in proximity to the cathode rebar 51-1.

The ECE system 401 further comprises an electrolyte 440. The electrolyte 440 is provided as a conductive medium between the anode 420 (for example titanium mesh anode) and the concrete surface 58. The electrolyte 440 is configured to support ion migration by creating a consistent ionic pathway between the anode 420 and the concrete surface 58. The electrolyte 440 is provided to support uniform current distribution. The electrolyte 440 may comprise a high-conductivity liquid electrolyte such as calcium hydroxide within a foam spray, a cementitious paste, or a conductive gel designed for extended treatments. The electrolyte 440 may be provide in a layer 441 between the anode 420 and the concrete surface 58.

The ECE system 401 further comprises an energy source 700 for powering the system 401. As described above power may be provided by a battery or other suitable source. In the exemplary a PV power source 720 is provided for powering the ECE system 401. The PV power source 720 advantageously provides a sustainable energy source without the need for battery storage. The PV power source 720 comprises PV panels 721 which are configured to convert solar energy into direct current (DC) electricity.

The ECE system 401 is configured to operate intermittently. This intermittent operation configuration effectively supports the ECE treatment process 460 which is based on a gradual chloride diffusion. The ECE system 401 is configured to operate intermittently in alignment with sunlight availability as powered by the PV power source. The arrangement of ECE system 401 advantageously eliminates the need for energy storage at the target site. The control unit 230 of the local system 200 is configured to regulate the power to ensure consistent voltage and current application during treatment. The control unit 230 further comprises a power regulator 241.

As noted above, the ECE treatment system 401 is provided in modular form as remediation module 400 of the locally installed system 200. The ECE treatment system 401 in modular form 400 may be installed in a retro-fitting installation at the target location 50 and is configured for coupling and interfacing directly with the system 200 and the wider system and network 100, 500, 600. As described above the control unit 230 of the system 200 provides for control of the connected modules which are configured to be coupled thereto in a plug and play arrangement.

The control unit 230 of the system 200 is configured to regulate voltage and current applied across the anode 420 and cathode 430 during ECE treatment 460 based on real-time sensor feedback from the monitoring module 300.

Data analysis of data measurements obtained by the monitoring module 300 and control may be provided locally at the system 200 and/or based on control provided by the systems 500, 600.

The control unit 230 may further comprises a power regulator 241, a controller 243 for control of connected modules, and a voltage and current supply controller 245 for control and regulation of voltage and current applied to the anode 420 and cathode 430. The control unit 230 may further comprise a data analyser 248.

In the exemplary arrangements of the specification, the control unit 230 is configured to monitor half-cell potential 371, chloride ion concentration 351, and resistivity values 361 for use in dynamically adjusting the current 480 applied to the anode 420 and ensure efficient chloride extraction 465 while preventing over-treatment. Monitoring may be provided on an intermittent basis. Monitoring may be provided continuously.

As an example, a typical ECE treatment 460 may be provides based on operation of the system 401 within a voltage range of 12V to 24V DC and a current density range of approximately 1 to 5 A/m², depending on structural conditions and chloride contamination severity.

The control unit 230 receives 260 data 261 from the sensors 327 of the sensor probes 301 locally for example by wireless communication, such as for example Bluetooth or WiFi. The control unit 230 transmits 263 data 261 via a communication system for example LoRa long range communication to the data management and control system 500.

In an example arrangement, the system 500 may be implemented as a cloud server system for long-term data storage and analysis.

Example methods of installation and of retro-fitting installation of a monitoring module and a remediation module are described further with reference to Figures 5A, 5B and 5C. Reference is made also to Figures 6 and 7.

The system 200 may be installed including the monitoring module 300 and remediation modules 400 or the monitoring module 300 only.

The system 100 is configured to effective deployment in both newly constructed and existing reinforced concrete structures. Placement of the sensor probes of the monitoring module is strategically determined based on corrosion risk zones identified through structural analysis to ensure comprehensive coverage. Key considerations for installation include:
- Accessibility for retrofitting within existing infrastructures.
- Long-term exposure conditions, such as marine and high-humidity environments. Strategic placement near critical structural elements, including high-stress zones

An exemplary installation method 600 for installation of the monitoring module and/or oneor more sensor probe includes one or more of the following:
1. Drilling 610 a core: A cylindrical core 54 also referred to as a concrete core void 54 is drilled. The core void 54 is created having a diameter sufficient to house the sensor probe 301. The core 54 is drilled into the concrete 50. The core 54 is located adjacent to a steel reinforcement 51 to ensure precise corrosion monitoring 303.
   FIGS 6A, 6B and 6C (and Figures 7A) show an arrangement in which a plurality of cores 54 (also referred to as core voids 54) are drilled from the concrete, to allow placement of the sensor probes 301 adjacent to the rebar 51.
2. Determining 615 sensor locations. Sensors 327 of the sensor probes 301 are located at predetermined locations relative to critical structural elements 51 which are considered necessary areas for monitoring 303.
3. Installing 620 sensor probes 301. The sensor probe unit 301 are inserted into the core such that the measurement interface of various sensors 327 are aligned correctly i.e. as required relative to the steel reinforcement 51 in the concrete structure. (Figure 2-1 (c)).
4. Connecting 625 the rebar to the sensor probe 301. A small pilot hole is drilled to the rebar 51 and a connection is made between the rebar 51 and the sensor probe 301. The connecting 625 may comprise attaching a wire between the rebar and sensor probe 301 at the port 380. A small trench may be formed between the sensor probe and a pilot hole location on the concrete surface 58. The wire 381 is then connected back to the sensor 301 and to port 380.
5. Initialisation 630 and Calibration 635. The sensor probe 301 is initialised, and baseline readings are verified.
6. Sealing 640 and Protecting 645: The sensor 301 core is securely sealed by drilling the cap onto the surface 58-2 at apertures 314. A sealant 318 is used between the cap 310 and the concrete surface 58-2 to prevent environmental interference while allowing future access if needed.

The installation method 600 may further comprise installation of the remediation module 400 including one or more of the following:
1. Locating 650 the anode 420. Locating and applying a mesh anode 420 (for example a titanium mesh anode) to a concrete surface 58 where treatment is required (Figure 2-2
   (a).). It is then connected 651 to the control unit 230 (Figure 2-2 (b)).
2. Applying 655 the Electrolyte 440: A cementitious paste or conductive gel comprising an electrolyte is applied over the anode 420 to ensure consistent ionic contact between the anode 420 and the concrete surface 58.
3. Connecting 660 the Rebar 51: The embedded steel reinforcement 51-1 is used as the cathode 430 of the system 401. A connection is established between the steel rebar 51-1 and the control unit 230.
4. Connecting 670 a power supply: The ECE system 401 is configured to be powered by PV panels 720 connected directly to the control unit 230, ensuring a sustainable energy source for treatment 460.
5. Initialising 675 the system 200, 400: The control unit 230 initiates the ECE treatment 460, regulating the current and voltage applied across the anode 420 and cathode 430 based on feedback from the monitoring module 300.
6. Monitoring 680 and Optimising 685: Real-time data from the sensors informs the control unit to adjust treatment intensity and duration dynamically
7. Linking 690 to the Control Unit:
   - Each wireless sensor transmits data directly to the control unit.
   - Upon initialisation, sensors are automatically detected by the control unit for data logging and treatment control.
   - The control unit assigns each sensor a unique identifier for data correlation and treatment zone mapping.
   - The control unit also regulates and monitors the ECE system, adjusting parameters based on sensor feedback.

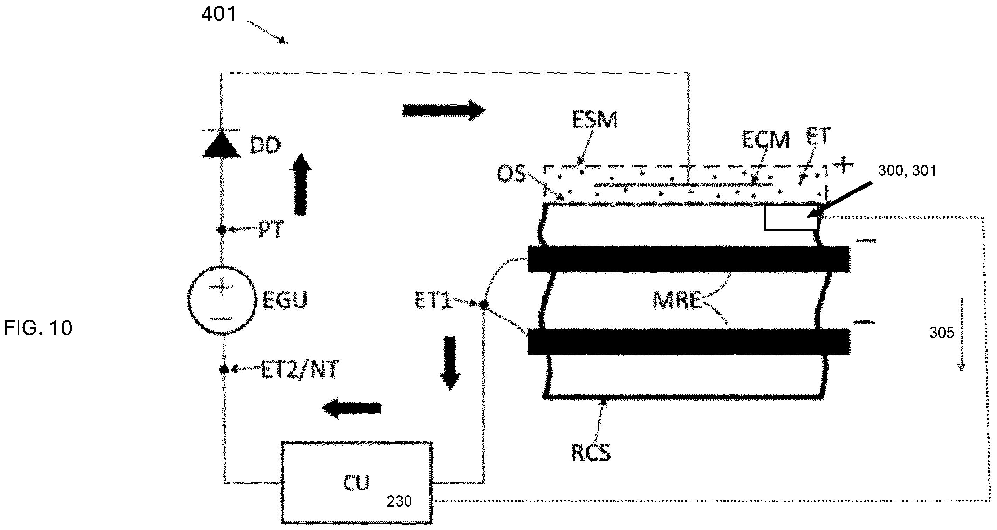

Referring to Figure 10 an exemplary arrangement of an ECE system installation is shown. The ECE system 401 is coupled to a monitoring module 300 and/or sensor probe 301 via control unit 230. One or more sensors 301 provide data 305 to the control unit for use in control of operation of the ECE 401.

Further methods for installation include the following:
The installation which may comprise a retro-fitting installation includes the following:
- Selecting a target location 50 for installation of the system 200.
- Removing a core plug from the concrete at a target location that is a close to a rebar. Creating a core void 54. The core void 54 located at a separation d1 from the rebar. The separation d1 is within a predefined distance range of 10-30 mm. In one exemplary arrangement the sensor probe may be located for example around 20mm from the rebar. The core void 54 is created at an orientation transverse to the rebars in the concrete so as to receive and to locate the sensors at a transverse orientation to the rebars. The core void 54 is formed having a diameter and longitudinal extent configured to receive a sensor probe 301 of the monitoring module 300. In the exemplary arrangement of Figures 2, three core voids 54-1, 54-2, 54-3 are formed to receive three sensor probes 301-1, 301-2 and 301-3 each spaced apart from each other and each being located at a required separation from a rebar and at a required orientation relative to the rebar.
- Installing sensor probe 301 in the corresponding core void 54. The sensor probe 301 as described in further detail below is configured for a friction fit within the core void 54. The sensor probe 301 comprises keying features 312 to allow precise control of the orientation of the sensor module relative to the rebar at the required orientation. The sensor probe comprises sealing and locking features to allow sealing of the sensor probe 301 within the core void.
- Installing the control unit 230. The control unit 230 is installed coupled to the monitoring module 300. The control unit 230 is configured to receive data 305 from the sensor module and to communicate that data to the data and control system 500.

The system 200 and the monitoring module 300 are configured for retro-fitting at a target location at a building or concrete structure. The method of installation requires identification of the rebar 51 locations at the target 50 and the creation of core voids 54 for receiving the sensor probes 301. The senor probes 301/monitoring module 300 and the system 200 are configured for plug and play and to be weatherproof and for continued operation at a range of temperatures and in conditions that vary with climate and weather. The on-site installation has low power requirements and is configured to communicate with the data and control system and for remote control. Each of the modules 300, 400 of the system 200 are self-contained modules which are configured for plug and play coupling within the system. This is advantageous taking account that the system 200 may be installed at a location with limited access - for example underground or at an elevated target site.

The remediation module 400 may be installed concurrently with the monitoring module. Alternatively, the remediation module 400 may be installed later if the monitoring indicates that remediation treatment is required. It will also be appreciated that the remediation module may be installed but activated later if the monitoring indicates that remediation treatment is required. Similarly, the remediation module is removable without affecting the operation of the other components - so that the remediation module may be removed while monitoring is maintained or may be deactivated while monitoring is maintained. The modular arrangement configured for

After treatment if remediation is no longer needed, it would also be possible to decouple the remediation module 400 from the monitoring module 300 and the locally installed system 200 and remove, which maintaining the monitoring module 300 in location for continuing monitoring if needed.

Operation of the system includes the following:

### 1 Timing with Respect to PV Operation and Free/Bound Chlorides Efficiency

The ECE system 401 is powered or supplemented by one or more photovoltaic (PV) panels 720. The use of solar-derived power naturally introduces intermittent operation, particularly cycling between daytime (power available) and nighttime (power off or minimal). Such intermittent operation has been found to enhance chloride extraction efficiency due to the presence of both free and bound chlorides within the concrete matrix. When the electrochemical remediation (ECE) process 460 is paused (e.g., overnight), bound chlorides in the cement paste partially re-equilibrate and convert into the free (ionic) form. This reequilibration effectively resets or increases the available fraction of free chlorides that can be driven out by the next active treatment cycle. As a result, repeating cycles of power-on/power-off correlated with sunlight availability can, in certain cases, improve the total chloride removal over a given treatment period while reducing the need for continuous power or large battery banks.

Referring to Table 1 below, the operations of the monitoring module 300 and the use and analysis of the sensor data 305 according to the invention is described further. In particular, the sensor probe 301 of the monitoring module comprises a plurality of sensor 327 which are operable in co-operation to provide the measurement data 305 that underpins the monitoring operations 303 and that is used to provide a responsive and precision control of the treatment operation 460.

**TABLE 1 - Parameter-Interaction Matrix**

| | Half-Cell Potential (HCP) 370 | 4-Point Wenner Resistivity (WR) 360 | Chloride Ion Concentration (Cl⁻) 350 | Temperature (T) 340 |
|---|---|---|---|---|
| Half-Cell Potential (HCP) 370, data 375 | - | HCP ↔ WR: Changes in moisture/resistivity can affect HCP readings (wet concrete can mask corrosion signals). | HCP ↔ Cl⁻: Correlation between potential and chloride thresholds (low HCP often indicates high chloride risk). | HCP ↔ T: Potential measurements depend on temperature corrections (electrode responses vary with T). |
| 4-Point Wenner Resistivity (WR) 360, data 365 | WR ↔ HCP: Resistivity data help confirm if negative half-cell values are due to genuine corrosion or just moisture. | - | WR ↔ Cl⁻: High chloride typically correlates with lower resistivity, especially in saturated concrete. | WR ↔ T: Resistivity is temperature-dependent (higher T generally lowers resistivity). |
| Chloride Ion Concentration (Cl⁻) 350, data 355 | Cl⁻ ↔ HCP: Chlorides break down the passivating layer, directly reflected in more negative half-cell potentials. | Cl⁻ ↔ WR: Elevated chloride can reduce resistivity, making it a complementary metric for diagnosing chloride-induced corrosion zones. | | Cl⁻ ↔ T: Diffusion rates and electrode response are temperature-dependent (warm conditions accelerate Cl⁻ movement). |
| Temperature (T) 340, data 345 | T ↔ HCP: Reference electrode potentials shift with temperature, requiring compensations. | T ↔ WR: Resistivity changes with temperature, impacting how moisture is interpreted. | T ↔ Cl⁻: Ion mobility and sensor output drift with changing temperature, requiring calibration. | - |

The system 100 and methods of monitoring 303
- Each cell of Table 1 shows how the measurement in the row (based on the specific sensor and the data measured) affects, or is affected by, the measurement in the column (based on the specific sensor and the data measured).
- The system and method of the exemplary arrangement of the present specification provide improved synergy based on the combination of sensors and sensor measurement data. For example, Chloride Ion Concentration directly influences Half-Cell Potential (because corrosion risk rises with more chloride), but that same chloride presence also lowers Resistivity, which can feed back into how the system interprets HCP data.
- Temperature cuts across all sensors of the sensor probes 301 and monitoring module 300, as every electrochemical reading (HCP, resistivity, chloride concentration) will shift with temperature.

### Sensor Data Flow Model

### Multi-Sensor Inputs:

In the system 100 (data analysis system 500 and or data analysis at local system 200) Half-Cell Potential 375, Resistivity 365, Chloride Concentration 355, and Temperature 345 data all get combined and adjusted for cross-influences (e.g., temperature corrections, moisture/resistivity interplay, chloride thresholds).

The combined sensor data 305 comprises data from the various sensors of the sensor probe 301.

### Data Aggregation & Machine Learning

A local microcontroller (system 200 and/or sensor probe microcontroller 337 (and/or the cloud-based system 500) merges sensor reading data 305, runs noise filtering, and leverages predictive analytics to detect early corrosion signs or forecast future issues.

### ECE Control Unit 230

If sensor data 305 indicates high corrosion risk (e.g., negative HCP, low resistivity, high chloride, rising temperature accelerating corrosion), the control unit 230 activates or adjusts the ECE process.

The ECE system 401 (powered by PV 720) is operated to drive chloride ions away from the rebar 51-1.

The sensors 327 of the monitoring module 300 continue monitoring 303 changes in chloride, potentials, and resistivity to confirm when the structure moves from active corrosion toward a more passive state. The changes may be monitored 303 in real-time. The changes may be monitored at intervals or continuously. Some of the sensors of the plurality of sensor may be configured to operate at particular intervals or continuously, as required.

### Feedback Loop

As measurements normalize (e.g., HCP becomes less negative, Cl⁻ drops below thresholds), the system dials down or suspends ECE to avoid over-treatment. This cyclical feedback ensures energy efficiency and optimal remediation.

In one example implementation a plurality of devices 200-1, 200-2, 200-n may be installed at different locations on reinforced concrete structure for example a building. Each device may be set up to provide feedback to the central system 500. A user at a connected user device 600-1 would be able to review the data across the multiple devices installed at the building, and to implement a remediation if required based on the monitored data.

The system 100 overall comprising locally installed and remotely accessible components advantageously provides a structural health monitoring and automated response treatment system 100. The system 100 advantageously provides real-time monitoring 303 and proactive remediation 460 for reinforced concrete structures, including for example bridges, tunnels, marine infrastructure, and buildings.

The system comprises a monitoring module 300 that is configured to be installed locally at a target or test site. The monitor module 300 integrates a multi-sensor array 302, including half-cell potential sensors 370, concrete resistivity sensors 360, temperature sensors 340, and chloride ion concentration sensors 350. The sensor probe 301 in a preferred arrangement is battery-operated and wireless, ensuring flexible installation without the need for external power sources or cabling. The sensor probe 301 is configured to be retrofittable to existing structures and accessible after installation, allowing for easy maintenance and upgrades, and designed with a rugged, waterproof casing to ensure reliable operation in harsh environments, including marine and high-humidity conditions.

Within the system 100 data transmission can be performed via any suitable communications protocol. For example, a LoRa long-range radio wave communication may be provided between the local system 200 located at a target site and a cloud server 500 for processing. Communication may also be provided locally via Bluetooth or WiFi between module of the system 200 or between the system 200 and a mobile device 600. This flexible communication setup provides for reliable, data collection and accessibility, even in remote locations.

The sensed data 305 is provided for processing and analysis to provide data to support operation of the system 200 for continued monitoring 303 and/or remediation 460. The data 305 received from the on-site system 200 may further be proceeding using machine learning algorithms to forecast structural health conditions and provide predictive analysis of potential deterioration trends. The models are continuously updated as new data is collected, enhancing forecasting accuracy over time. This data-driven approach allows for early detection of structural issues, minimizing the risk of severe damage.

A user-friendly front-end interface may be provided to present and support user access to the data and to data analysis results and real-time data, enabling the end user to monitor structural health and receive alerts for necessary interventions. The interface simplifies data interpretation for engineers and maintenance teams.

In the event an issue or deterioration is detected based on the measured data 305 from a monitoring module 200, the system can activate an electrochemical chloride extraction (ECE) treatment 460. Chloride ion concentration measurements from the sensors are used to determine treatment duration and efficiency, ensuring optimal remediation. Half-cell potential readings guide the transition from active to passive corrosion states, indicating when the corrosion risk has been mitigated. The ECE treatment system is powered by photovoltaic (PV) panels, eliminating the need for diesel generators or battery storage. The ECE process can operate intermittently with direct PV power without the need for energy storage systems, further enhancing system sustainability and reducing complexity.

Further advantageous features include the following:
- Real-time structural health monitoring with embedded sensors.
- Automated corrosion remediation using electrochemical chloride extraction (ECE).
- Flexible wireless data transmission via LoRa, Bluetooth, or WiFi.
- Battery-operated, completely wireless sensors for flexible installation and long-term operation.
- Retrofits easily to existing structures and remains accessible post-installation.
- Rugged, waterproof sensor design suitable for harsh environments.
- Machine learning algorithms for predictive maintenance and anomaly detection.
- User-friendly front-end for real-time data visualization and alerts.
- PV-powered ECE treatment system eliminating diesel generator reliance.
- Secure data transmission and compliance with industry standards for structural monitoring.

It will be understood that the embodiments illustrated above show applications only for the purposes of illustration. In practice, embodiments may be applied to many different configurations, the detailed embodiments being straightforward for those skilled in the art to implement.

## Claims

1. A sensor probe (301) configured for use in monitoring the state of health of concrete structure, wherein the sensor probe (301) comprises:
a plurality of sensors (327, 341, 350, 360, 370);
a sensor probe body (320) configured in use to be received in a core void (54) formed in the reinforced concrete, at the test site;
wherein the sensor probe body supports the plurality of sensors (327, 341, 350, 360 and 370) to provide for the precise positioning of the sensors relative to the core void (54).

2. The sensor probe of claim 1, wherein the sensor probe is configured to be installed in a retro-fit installation at a target site.

3. The sensor probe of claim 1 or claim 2, further comprising:
a sensor probe cap (310);
wherein the sensor probe body (320) and cap (310) are arranged about a central longitudinal axis (Z);
wherein the sensor probe body (320) comprises a sensor probe housing (324) and an external surface (325), the sensor probe body (320) having an elongated form extending longitudinally (Z1-Z1) from a distal end (322) to a proximal end (321);
wherein the sensor probe cap (310) is located at the proximal end of the sensor probe and configured in use to be located externally of the core void (54);
wherein the sensor probe body (320) and the cap (310) are configured to provide for precise positioning of each sensor (327, 341, 350, 360 and 370) relative to the core void.

4. The sensor probe of any preceding claim, wherein the sensor probe (301) comprises:
at least one potential sensor (370) to measure the electrochemical potential difference between the embedded concrete steel reinforcement (51) and a reference electrode (373, 1373), to detect corrosion activity.

5. The sensor probe of claim 4,
wherein the potential sensor (370) comprises a Half-Cell potential sensor;
wherein the reference electrode comprises a stable half-cell electrode, for example: an Ag/AgCl reference electrode (373) or Cu/CuSO4 reference electrode (373); and
wherein the potential sensor (370) is operable to measure the potential difference generated by chloride ion activity compared to the reference electrode.

6. The sensor probe of any preceding claim, wherein the sensor probe (301) comprises a resistivity sensor (360) configured to measure concrete resistivity to assess moisture and ion mobility.

7. The sensor probe of claim 6, wherein the resistivity sensor (360) comprises a 4-Point Wenner Probe (1360) comprising four electrodes (361) to measure the electrical resistivity of the concrete.

8. The sensor probe of claim 5, wherein the sensor probe (301) comprises a chloride sensor (350), wherein the chloride sensor is Chloride Ion-Selective Electrode, Cl ISE, sensor configured to measure chloride ion concentration for corrosion risk assessment and to monitor for chloride reduction during ECE treatment.

9. The sensor probe of any preceding claim, wherein the sensor probe (301) comprises one or more of:
(i) a temperature sensor (340) to measure ambient and internal concrete temperature to allow for adjustment of corrosion and resistivity measurements;
(ii) a temperature and humidity sensor (340) to measure ambient and internal concrete temperature and humidity to allow for adjustment of corrosion and resistivity measurements.

10. The sensor probe of any preceding claim,
wherein sensor probe (301) is configured detect data (305) indicative of state of health (304) of the reinforced concrete at the test site;
wherein the sensor probe (301) comprises a plurality of sensors (327, 350, 340, 360, 370) each configured to sense different parameters and properties to support a comprehensive analysis of the state of health (304) of the reinforced concrete at the test site; and
wherein the detected data (305) includes data measured by a plurality of sensors (327, 355, 345, 365, 375).

11. The sensor probe of any preceding claim, wherein the sensor probe cap (310) comprises a fixing member (314) for fixing the sensor probe in location at the test site, and a sealing member (317) for sealing the sensor probe located in the core void (54) from an external environment

12. The sensor probe of any preceding claim, wherein the sensor probe cap (310) comprises a keying element (312) defining a visual indicator to guide a user to precise location of the sensors at a required depth and/or angular orientation relative to the core void (54) and any structural elements embedded in the concrete structure.

13. The sensor probe of any preceding claim,
wherein the sensor probe (301) is configured to be coupled to an electrochemical chloride extraction, ECE, system (400), wherein the sensor probe (301) is configured to provide data (305) for use in identifying corrosion and in controlling the operation of the ECE system (400);
wherein the sensed data includes a plurality of data relating to different parameters as detected by the plurality of sensors, wherein the sensed data is indicative of the state of health of the concrete.

14. A system (100, 200) for monitoring for corrosion and treatment of corrosion in a reinforced concrete structure (40, 50, 51) at a target location, the reinforced concrete structure comprising one or more embedded reinforced bars (51), wherein the system comprises:
at least one sensor probe (301) as claimed in any of claims 1-13; and
a remediation module (400) comprising an electrochemical chloride extraction, ECE, system (401) configured to provide treatment and remediation (460) of corrosion at the target location, wherein the ECE system (401) comprises an anode and a cathode, wherein the cathode is defined by an embedded reinforced bar of the concrete structure; and
a controller (230, 500);
wherein the at least one sensor probe (301) is configured to sense data (305) indicative of the state of health of the concrete and, and to provide the sensed data (305) a controller (230, 500);
wherein the controller (230, 500) is configured to receive sensed data (305) and to analyse the data (305) and determine whether remediation action is required; and
wherein, upon determining based on the sensed data (305) that remediation action is required, the controller (230, 500) is configured to activate the ECE system (401) to deliver remediation treatment (460).

15. The system of claim 14, wherein operation of the ECE system (401) is controllable based on feedback (305) from the one or more sensor probes (301), wherein treatment intensity and duration are dynamically adjustable based on the feedback (305).
